(19) Europäisches Patentamt
European Patent Office
Office européen des brevets

(11) EP 2 928 896 B1

(12) # EUROPEAN PATENT SPECIFICATION

(45) Date of publication and mention of the grant of the patent:
**19.07.2017 Bulletin 2017/29**

(21) Application number: **13824177.3**

(22) Date of filing: **26.11.2013**

(51) Int Cl.:
***C07D 498/04*** *(2006.01)* ***A61K 31/5365*** *(2006.01)*
***A61P 29/00*** *(2006.01)*

(86) International application number:
**PCT/IB2013/060412**

(87) International publication number:
**WO 2014/102630 (03.07.2014 Gazette 2014/27)**

(54) **SOLID FORM OF DIHYDRO-PYRIDO-OXAZINE DERIVATIVE**

FESTE FORM EINES DIHYDRO-PYRIDO-OXAZIN-DERIVATS

FORME SOLIDE D'UN DÉRIVÉ DE DIHYDRO-PYRIDO-OXAZINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.11.2012 GC P201222895**

(43) Date of publication of application:
**14.10.2015 Bulletin 2015/42**

(73) Proprietor: **NOVARTIS AG**
**4056 Basel (CH)**

(72) Inventors:
- **HURTH, Konstanze**
**CH-4002 Basel (CH)**
- **KALIS, Christoph**
**CH-4002 Basel (CH)**
- **KAMMERTOENS, Karen**
**CH-4002 Basel (CH)**
- **SOLDERMANN, Nicolas**
**CH-4002 Basel (CH)**
- **ZECRI, Frédéric**
**Cambridge, Massachusetts 02139 (US)**

(74) Representative: **Marti, Christiane**
**Novartis Pharma AG**
**Patent Department**
**4002 Basel (CH)**

(56) References cited:
**WO-A1-2013/093849**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 928 896 B1

## Description

FIELD OF THE INVENTION

The invention relates to a novel solid form of a dihydro-pyrido-oxazine derivative, processes for its preparation and its use in pharmaceutical compositions.

BACKGROUND OF THE INVENTION

The international patent application PCT/IB2012/057554, published as WO2013/093849 discloses dihydro-benzo-oxazine and dihydro-pyrido-oxazine derivatives that are suitable for the treatment of a disorder or disease which is mediated by the activity of the PI3K enzymes. PCT/IB2012/057554, discloses (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and methods of making this compound.

These compounds are useful for the treatment, either alone or in combination, with one or more other pharmacologically active compounds, of PI3K-related diseases including but not limited to autoimmune disorders, inflammatory diseases, allergic diseases, airway diseases, such as asthma and COPD, transplant rejection, cancers eg of hematopoietic origin or solid tumors.

These compounds are also useful for treatment, either alone or in combination, with one or more other pharmacologically active compounds, of conditions, diseases or disorders for example autoimmune disorders, inflammatory diseases, allergic diseases, airway diseases, such as asthma and COPD, transplant rejection; antibody production, antigen presentation, cytokine production or lymphoid organogenesis are abnormal or are undesirable including rheumatoid arthritis, pemphigus vulgaris, idiopathic thrombocytopenia purpura, systemic lupus erythematosus, multiple sclerosis, myasthenia gravis, Sjögren's syndrome, autoimmune hemolytic anemia, ANCA-associated vasculitides, cryoglobulinemia, thrombotic thrombocytopenic purpura, chronic autoimmune urticaria, allergy (atopic dermatitis, contact dermatitis, allergic rhinitis), goodpasture's syndrome, AMR (antibody-mediated transplant rejection), B cell-mediated hyperacute, acute and chronic transplant rejection and cancers of haematopoietic origin including but not limited to multiple myeloma; a leukaemia; acute myelogenous leukemia; chronic myelogenous leukemia; lymphocytic leukemia; myeloid leukemia; non-Hodgkin lymphoma; lymphomas; polycythemia vera; essential thrombocythemia; myelofibrosis with myeloid metaplasia; and Walden stroem disease; more suitably from rheumatoid arthritis (RA), pemphigus vulgaris (PV), idiopathic thrombocytopenia purpura (ITP), thrombotic thrombocytopenic purpura (TTP), autoimmune hemolytic anemia (AIHA), acquired hemophilia type A (AHA), systemic lupus erythematosus (SLE), multiple sclerosis (MS), myasthenia gravis (MG), Sjögren's syndrome (SS), ANCA-associated vasculitides, cryoglobulinemia, chronic autoimmune urticaria (CAU), allergy (atopic dermatitis, contact dermatitis, allergic rhinitis), goodpasture's syndrome, transplant rejection and cancers of haematopoietic origin as well as in disease or infection associated immunopathology, for example in severe and cerebral malaria, trypanosomiasis, leishmaniasis, toxoplasmosis and neurocysticercosis.

SUMMARY OF THE INVENTION

The invention relates an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone; pharmaceutical compositions and combinations including this form. The invention further relates this form, including its pharmaceutical compositions and combinations for use in the treatment of diseases mediated by the activity of the PI3K enzymes, preferably by the activity of the PI3Kδ isoform.

It is well known that the crystalline form of the active pharmaceutical ingredient (API) of a particular drug is often an important determinant of the drug's ease of preparation, hygroscopicity, stability, solubility, storage stability, ease of formulation, rate of dissolution in gastrointestinal fluids and in vivo bioavailability. Crystalline forms occur where the same composition of matter crystallizes in a different lattice arrangement resulting in different thermodynamic properties and stabilities specific to the particular crystalline form. Crystalline forms may also include different hydrates or solvates of the same compound. In deciding which form is preferable, the numerous properties of the forms are compared and the preferred form chosen based on the many physical property variables. It is entirely possible that one form can be preferable in some circumstances where certain aspects such as ease of preparation, stability, etc. are deemed to be critical. In other situations, a different form may be preferred for greater dissolution rate and/or superior bioavailability. It is not yet possible to predict whether a particular compound will form polymorphs, whether any such polymorphs will be suitable for commercial use in a therapeutic composition, or which polymorphs will display such desirable properties.

BRIEF DESCRIPTION OF THE DRAWINGS

**Figure 1** is the X-ray Powder Diffraction Pattern of Example F1, crystalline anhydrous form
**Figure 2** is the differential scanning calorimetry graph of Example Example F1, crystalline anhydrous form

DETAILED DESCRIPTION OF THE INVENTION

In one embodiment, the invention relates to an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone.

In another embodiment, the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone is characterized by an X-Ray powder diffraction pattern comprising the following peaks given at degrees 2-Theta +/- 0.2 degrees: 9.1, 10.2, 11.9, 13.0, 17.1, 17.7, 18.7, 20.3, 20.8, 26.0, 26.7, 23.2, 24.1, 24.8, 29.3, 27.4, and 21.4.

In another embodiment, the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone is having a X-ray diffraction spectrum substantially the same as the X-ray powder diffraction spectrum shown in Figure 1.

In another embodiment, the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone is having a differential scanning calorimetry graph substantially the same as that shown in shown in Figure 2.

Unless otherwise specified, the term "form of the present invention" refers to an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone.

The general terms used hereinbefore and hereinafter preferably have within the context of this disclosure the following meanings, unless otherwise indicated:

The invention may be more fully appreciated by reference to the following description, including the following glossary of terms and the concluding examples. As used herein, the terms "including", "containing" and "comprising" are used herein in their open, non-limiting sense.

As used herein, the term "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drug stabilizers, binders, excipients, disintegration agents, lubricants, sweetening agents, flavoring agents, dyes, and the like and combinations thereof, as would be known to those skilled in the art (see, for example, Remington's Pharmaceutical Sciences, 18th Ed. Mack Printing Company, 1990, pp. 1289- 1329). Except insofar as any conventional carrier is incompatible with the active ingredient, its use in the therapeutic or pharmaceutical compositions is contemplated.
The term "a therapeutically effective amount" of a compound of the present invention refers to an amount of the compound of the present invention that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of the compound of the present invention that, when administered to a subject, is effective to (1) at least partially alleviate, inhibit, prevent and/or ameliorate a condition, or a disorder or a disease (i) mediated by PI3K or (ii) associated with PI3K activity, or (iii) characterized by activity (normal or abnormal) of PI3K or (2) reduce or inhibit the activity of PI3K or (3) reduce or inhibit the expression of PI3K. In another non-limiting embodiment, the term "a therapeutically effective amount" refers to the amount of the compound of the present invention that, when administered to a cell, or a tissue, or a non-cellular biological material, or a medium, is effective to at least partially reducing or inhibiting the activity of PI3K; or at least partially reducing or inhibiting the expression of PI3K. The meaning of the term "a therapeutically effective amount" as illustrated in the above embodiment for PI3K also applies by the same means to any other relevant proteins/peptides/enzymes.

As used herein, the term "subject" refers to an animal. Typically the animal is a mammal. A subject also refers to for example, primates (*e.g.*, humans, male or female), cows, sheep, goats, horses, dogs, cats, rabbits, rats, mice, fish, birds and the like. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers in one embodiment,

to ameliorating the disease or disorder (i.e., slowing or arresting or reducing the development of the disease or at least one of the clinical symptoms thereof). In another embodiment "treat", "treating" or "treatment" refers to alleviating or ameliorating at least one physical parameter including those which may not be discernible by the patient. In yet another embodiment, "treat", "treating" or "treatment" refers to modulating the disease or disorder, either physically, (*e.g.*, stabilization of a discernible symptom), physiologically, (*e.g.*, stabilization of a physical parameter), or both. In yet another embodiment, "treat", "treating" or "treatment" refers to preventing or delaying the onset or development or progression of the disease or disorder.

As used herein, a subject is "in need of" a treatment if such subject would benefit biologically, medically or in quality of life from such treatment.

All methods described herein can be performed in any suitable order unless otherwise indicated herein or otherwise clearly contradicted by context. The use of any and all examples, or exemplary language, *e.g.* "such as", provided herein is intended merely to better illuminate the invention and does not pose a limitation on the scope of the invention otherwise claimed.

In another aspect, the present invention provides a pharmaceutical composition comprising a form of the present invention and a pharmaceutically acceptable carrier. The pharmaceutical composition can be formulated for particular routes of administration such as oral administration, parenteral administration, and rectal administration, etc. In addition, the pharmaceutical compositions of the present invention can be made up in a solid form (including without limitation capsules, tablets, pills, granules, powders or suppositories), or in a liquid form (including without limitation solutions, suspensions or emulsions). The pharmaceutical compositions can be subjected to conventional pharmaceutical operations such as sterilization and/or can contain conventional inert diluents, lubricating agents, or buffering agents, as well as adjuvants, such as preservatives, stabilizers, wetting agents, emulsifers and buffers, etc.
Typically, the pharmaceutical compositions are tablets or gelatin capsules comprising the active ingredient together with

a) diluents, *e.g.*, lactose, dextrose, sucrose, mannitol, sorbitol, cellulose and/or glycine;
b) lubricants, *e.g.*, silica, talcum, stearic acid, its magnesium or calcium salt and/or polyethyleneglycol; for tablets also
c) binders, *e.g.*, magnesium aluminum silicate, starch paste, gelatin, tragacanth, methylcellulose, sodium carboxymethylcellulose and/or polyvinylpyrrolidone; if desired
d) disintegrants, *e.g*., starches, agar, alginic acid or its sodium salt, or effervescent mixtures; and/or
e) absorbents, colorants, flavors and sweeteners.

Tablets may be either film coated or enteric coated according to methods known in the art.

Suitable compositions for oral administration include an effective amount of a form of the present invention in the form of tablets, lozenges, aqueous or oily suspensions, dispersible powders or granules, emulsion, hard or soft capsules, or syrups or elixirs. Compositions intended for oral use are prepared according to any method known in the art for the manufacture of pharmaceutical compositions and such compositions can contain one or more agents selected from the group consisting of sweetening agents, flavoring agents, coloring agents and preserving agents in order to provide pharmaceutically elegant and palatable preparations. Tablets may contain the active ingredient in admixture with nontoxic pharmaceutically acceptable excipients which are suitable for the manufacture of tablets. These excipients are, for example, inert diluents, such as calcium carbonate, sodium carbonate, lactose, calcium phosphate or sodium phosphate; granulating and disintegrating agents, for example, corn starch, or alginic acid; binding agents, for example, starch, gelatin or acacia; and lubricating agents, for example magnesium stearate, stearic acid or talc. The tablets are uncoated or coated by known techniques to delay disintegration and absorption in the gastrointestinal tract and thereby provide a sustained action over a longer period. For example, a time delay material such as glyceryl monostearate or glyceryl distearate can be employed. Formulations for oral use can be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example, peanut oil, liquid paraffin or olive oil.

Certain injectable compositions are aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Said compositions are prepared according to conventional mixing, granulating or coating methods, respectively, and contain about 0.1-75%, or contain about 1-50%, of the active ingredient.

Suitable compositions for transdermal application include an effective amount of a form of the present invention with a suitable carrier. Carriers suitable for transdermal delivery include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound of the skin of the host at a controlled and predetermined rate over a prolonged period

of time, and means to secure the device to the skin.

Suitable compositions for topical application, *e.g.*, to the skin and eyes, include aqueous solutions, suspensions, ointments, creams, gels or sprayable formulations, *e.g.*, for delivery by aerosol or the like. Such topical delivery systems will in particular be appropriate for dermal application, *e.g.*, for the treatment of skin cancer, *e.g.*, for prophylactic use in sun creams, lotions, sprays and the like. They are thus particularly suited for use in topical, including cosmetic, formulations well-known in the art. Such may contain solubilizers, stabilizers, tonicity enhancing agents, buffers and preservatives.

As used herein a topical application may also pertain to an inhalation or to an intranasal application. They may be conveniently delivered in the form of a dry powder (either alone, as a mixture, for example a dry blend with lactose, or a mixed component particle, for example with phospholipids) from a dry powder inhaler or an aerosol spray presentation from a pressurised container, pump, spray, atomizer or nebuliser, with or without the use of a suitable propellant.

The present invention further provides anhydrous pharmaceutical compositions and dosage forms comprising the form of the present invention as active ingredients, since water may facilitate the degradation of certain compounds.

Anhydrous pharmaceutical compositions and dosage forms of the present invention can be prepared using anhydrous or low moisture containing ingredients and low moisture or low humidity conditions. An anhydrous pharmaceutical composition may be prepared and stored such that its anhydrous nature is maintained. Accordingly, anhydrous compositions are packaged using materials known to prevent exposure to water such that they can be included in suitable formulary kits. Examples of suitable packaging include, but are not limited to, hermetically sealed foils, plastics, unit dose containers (*e. g.*, vials), blister packs, and strip packs.

The invention further provides pharmaceutical compositions and dosage forms that comprise one or more agents that reduce the rate by which the compound of the present invention as an active ingredient will decompose. Such agents, which are referred to herein as "stabilizers," include, but are not limited to, antioxidants such as ascorbic acid, pH buffers, or salt buffers, etc.

A form of the present invention may be useful in the treatment of conditions, diseases or disorders including disease or infection associated immunopathology in which one or more of the functions of B cells such as antibody production, antigen presentation, cytokine production or lymphoid organogenesis are abnormal or are undesirable including rheumatoid arthritis, pemphigus vulgaris and related diseases, idiopathic thrombocytopenia purpura, systemic lupus erythematosus, multiple sclerosis, myasthenia gravis, Sjögren's syndrome, autoimmune hemolytic anemia, ANCA-associated vasculitides, cryoglobulinemia, thrombotic thrombocytopenic purpura, chronic autoimmune urticaria, allergy (atopic dermatitis, contact dermatitis, allergic rhinitis), goodpasture's syndrome, AMR (antibody-mediated transplant rejection), B cell-mediated hyperacute, acute and chronic transplant rejection and cancers of haematopoietic origin including but not limited to multiple myeloma; acute myelogenous leukemia; chronic myelogenous leukemia; lymphocytic leukemia; myeloid leukemia; non-Hodgkin lymphoma; lymphomas; polycythemia vera; essential thrombocythemia; myelofibrosis with myeloid metaplasia; and Walden stroem disease.

The invention includes treating conditions, diseases or disorders in which one or more of the functions of neutrophils, such as superoxide release, stimulated exocytosis, or chemoatractic migration are abnormal or are undesirable including rheumatoid arthritis, sepsis, pulmonary or resporatory disorders such as asthma, inflammatory dermatoses such as psoriasis, as well as in disease or infection associated immunopathology and others.

The invention includes treating conditions, diseases or disorders in which one or more of the functions of basophil and mast cells such as chemoatractic migration or allergen-IgE-mediated degranulation are abnormal or are undesirable including allergic diseases (atopic dermatitis, contact dermatitis, allergic rhinitis) as well as other disorders such as COPD, asthma or emphysema.

The invention includes treating conditions, diseases or disorders in which one or more of the functions of T cells such as cytokine production or cell-mediated cytotoxicity abnormal or are undesirable including rheumatoid arthritis, multiple sclerosis, acute or chronic rejection of cell tissue or organ grafts or cancers of haematopoietic origin as well as in disease or infection associated immunopathology.

Further, the invention includes treating neurodegenerative diseases, cardiovascular diseases and platelet aggregation.

Further, the invention includes treating skin diseases such as porphyria cutanea tarda, polymorphous light eruption, dermatomyositis, solar urticaria, oral lichen planus, panniculitis, scleroderma, urticarial vasculitis.

Further, the invention includes treating chronic inflammatory diseases such as sarcoidosis, granuloma annulare.

In other embodiments, the condition or disorder (e.g. PI3K-mediated) is selected from the group consisting of: polycythemia vera, essential thrombocythemia, myelofibrosis with myeloid metaplasia, asthma, COPD, ARDS, Loffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma, eosinophil-related disorders affecting the airways occasioned by drug-reaction, psoriasis, contact dermatitis, atopic dermatitis, alopecia areata, erythema multiforme, dermatitis herpetiformis, scleroderma, vitiligo, hypersensitivity angiitis, urticaria, bullous pemphigoid, lupus erythematosus, pemphigus, epidermolysis bullosa acquisita, autoimmune haematogical disorders (e.g. haemolytic anaemia, aplastic anaemia, pure red cell anaemia and idiopathic thrombocytopenia), systemic

lupus erythematosus, polychondritis, scleroderma, Wegener granulomatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary biliary cirrhosis, uveitis (anterior and posterior), interstitial lung fibrosis, psoriatic arthritis, glomerulonephritis, cardiovascular diseases, atherosclerosis, hypertension, deep venous thrombosis, stroke, myocardial infarction, unstable angina, thromboembolism, pulmonary embolism, thrombolytic diseases, acute arterial ischemia, peripheral thrombotic occlusions, and coronary artery disease, reperfusion injuries, retinopathy, such as diabetic retinopathy or hyperbaric oxygen-induced retinopathy, and conditions characterized by elevated intraocular pressure or secretion of ocular aqueous humor, such as glaucoma.

In another embodiment, a form of the present invention is useful in the treatment, prevention, or amelioration of autoimmune disease and of inflammatory conditions, in particular inflammatory conditions with an aetiology including an autoimmune component such as arthritis (for example rheumatoid arthritis, arthritis chronica progrediente and arthritis deformans) and rheumatic diseases, including inflammatory conditions and rheumatic diseases involving bone loss, inflammatory pain, spondyloarhropathies including ankolsing spondylitis, Reiter syndrome, reactive arthritis, psoriatic arthritis, and enterophathics arthritis, hypersensitivity (including both airways hypersensitivity and dermal hypersensitivity) and allergies. Specific auto-immune diseases for which forms of the invention may be employed include autoimmune haematological disorders (including e.g. hemolytic anaemia, aplastic anaemia, pure red cell anaemia and idiopa-thic thrombocytopenia), acquired hemophilia A, cold agglutinin disease, cryoglobulinemia, thrombotic thrombocytopenic purpura, Sjögren's syndrome, systemic lupus erythematosus, inflammatory muscle disorders, polychondritis, sclerodoma, antineutrophil cytoplasmic antibody- associated vasculitis, IgM mediated neuropathy, opsoclonus myoclonus syndrome, Wegener granulomatosis, dermatomyositis, chronic active hepatitis, myasthenia gravis, psoriasis, Steven-Johnson syndrome, pemphigus vulgaris, pemphigus foliacius, idio-pathic sprue, autoimmune inflammatory bowel disease (including e.g. ulcerative colitis, Crohn's disease and Irritable Bowel Syndrome), endocrine ophthalmopathy, Graves' disease, sarcoidosis, multiple sclerosis, neuromyelitis optica, primary biliary cirrhosis, juvenile diabetes (diabetes mellitus type I), uveitis (anterior, intermediate and posterior as well as panuveitis), keratoconjunctivitis sicca and vernal keratoconjunctivitis, interstitial lung fibrosis, psoriatic arthritis and glomerulonephritis (with and without nephrotic syndrome, e.g. including idiopathic nephro-tic syndrome or minimal change nephropathy), tumors, inflammatory disease of skin and cornea, myositis, loosening of bone implants, metabolic disorders, such as atherosclerosis, diabetes, and dislipidemia.

In another embodiment, a form of the present invention is useful in the treatment of conditions or disorders selected from the group consisting of, primary cutaneous B-cell lymphoma, immunobullous disease, pemphigus vulgaris, pemphigus foliaceus, endemic form of Brazilian pemphigus (Fogo selvagem), paraneoplastic pemphigus, bullous pemphigoid, mucous membrane pemphigoid, epidermolysis bullosa acquisita, chronic graft versus host disease, dermatomyositis, systemic lupus erythematosus, vasculitis, small vessel vasculitis, hypocomplementemic urticarial vasculitis, antineutrophil cytoplasmic antibody-vasculitis, cryoglobulinemia, Schnitzler syndrome, Waldenstrom's macroglobulinemia, angioedema, vitiligo, systemic lupus erythematosus, idiopathic thrombocytopenic purpura, multiple sclerosis, cold agglutinin disease, autoimmune hemolytic anemia, antineutrophil cytoplasmic antibody-associated vasculitis, graft versus host disease, cryoglobulinemia and thrombotic thrombocytopenic.

Thus, as a further embodiment, the present invention provides the use of an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone in therapy. In a further embodiment, the therapy is selected from a disease which may be treated by inhibition of PI3K. In another embodiment, the disease is selected from the aforementioned list, suitably from autoimmune disorders, inflammatory diseases, allergic diseases, airway diseases, such as asthma and COPD, transplant rejection; antibody production, antigen presentation, cytokine production or lymphoid organogenesis are abnormal or are undesirable including rheumatoid arthritis, pemphigus vulgaris, idiopathic thrombocytopenia purpura, systemic lupus erythematosus, multiple sclerosis, myasthenia gravis, Sjögren's syndrome, autoimmune hemolytic anemia, ANCA-associated vasculitides, cryoglobulinemia, thrombotic thrombocytopenic purpura, chronic autoimmune urticaria, allergy (atopic dermatitis, contact dermatitis, allergic rhinitis), goodpasture's syndrome, AMR (antibody-mediated transplant rejection), B cell-mediated hyperacute, acute and chronic transplant rejection and cancers of haematopoietic origin including but not limited to multiple myeloma; a leukaemia; acute myelogenous leukemia; chronic myelogenous leukemia; lymphocytic leukemia; myeloid leukemia; non-Hodgkin lymphoma; lymphomas; polycythemia vera; essential thrombocythemia; myelofibrosis with myeloid metaplasia; and Walden stroem disease; more suitably from rheumatoid arthritis (RA), pemphigus vulgaris (PV), idiopathic thrombocytopenia purpura (ITP), thrombotic thrombocytopenic purpura (TTP), autoimmune hemolytic anemia (AIHA), acquired hemophilia type A (AHA), systemic lupus erythematosus (SLE), multiple sclerosis (MS), myasthenia gravis (MG), Sjögren's syndrome (SS), ANCA-associated vasculitides, cryoglobulinemia, chronic autoimmune urticaria (CAU), allergy (atopic dermatitis, contact dermatitis, allergic rhinitis), goodpasture's syndrome, transplant rejection and cancers of haematopoietic origin as well as in disease or infection associated immunopathology, for example in severe and cerebral malaria, trypanosomiasis, leishmaniasis,

toxoplasmosis and neurocysticercosis.

In another embodiment, the invention provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in treating a disease which is treated by inhibition of PI3K. In a further embodiment, the disease is selected from the afore-mentioned list, suitably from autoimmune disorders, inflammatory diseases, allergic diseases, airway diseases, such as asthma and COPD, transplant rejection; antibody production, antigen presentation, cytokine production or lymphoid organogenesis are abnormal or are undesirable including rheumatoid arthritis, pemphigus vulgaris, idiopathic thrombocytopenia purpura, systemic lupus erythematosus, multiple sclerosis, myasthenia gravis, Sjögren's syndrome, autoimmune hemolytic anemia, ANCA-associated vasculitides, cryoglobulinemia, thrombotic thrombocytopenic purpura, chronic autoimmune urticaria, allergy (atopic dermatitis, contact dermatitis, allergic rhinitis), goodpasture's syndrome, AMR (antibody-mediated transplant rejection), B cell-mediated hyperacute, acute and chronic transplant rejection and cancers of haematopoietic origin including but not limited to multiple myeloma; a leukaemia; acute myelogenous leukemia; chronic myelogenous leukemia; lymphocytic leukemia; myeloid leukemia; non-Hodgkin lymphoma; lymphomas; polycythemia vera; essential thrombocythemia; myelofibrosis with myeloid metaplasia; and Walden stroem disease; more suitably from rheumatoid arthritis (RA), pemphigus vulgaris (PV), idiopathic thrombocytopenia purpura (ITP), thrombotic thrombocytopenic purpura (TTP), autoimmune hemolytic anemia (AIHA), acquired hemophilia type A (AHA), systemic lupus erythematosus (SLE), multiple sclerosis (MS), myasthenia gravis (MG), Sjögren's syndrome (SS), ANCA-associated vasculitides, cryoglobulinemia, chronic autoimmune urticaria (CAU), allergy (atopic dermatitis, contact dermatitis, allergic rhinitis), goodpasture's syndrome, transplant rejection and cancers of haematopoietic origin as well as in disease or infection associated immunopathology, for example in severe and cerebral malaria, trypanosomiasis, leishmaniasis, toxoplasmosis and neurocysticercosis.

Thus, as a further embodiment, the present invention provides the use of an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for the manufacture of a medicament. In a further embodiment, the medicament is for treatment of a disease which may be treated inhibition of PI3K. In another embodiment, the disease is selected from the afore-mentioned list, suitably from autoimmune disorders, inflammatory diseases, allergic diseases, airway diseases, such as asthma and COPD, transplant rejection; antibody production, antigen presentation, cytokine production or lymphoid organogenesis are abnormal or are undesirable including rheumatoid arthritis, pemphigus vulgaris, idiopathic thrombocytopenia purpura, systemic lupus erythematosus, multiple sclerosis, myasthenia gravis, Sjögren's syndrome, autoimmune hemolytic anemia, ANCA-associated vasculitides, cryoglobulinemia, thrombotic thrombocytopenic purpura, chronic autoimmune urticaria, allergy (atopic dermatitis, contact dermatitis, allergic rhinitis), goodpasture's syndrome, AMR (antibody-mediated transplant rejection), B cell-mediated hyperacute, acute and chronic transplant rejection and cancers of haematopoietic origin including but not limited to multiple myeloma; a leukaemia; acute myelogenous leukemia; chronic myelogenous leukemia; lymphocytic leukemia; myeloid leukemia; non-Hodgkin lymphoma; lymphomas; polycythemia vera; essential thrombocythemia; myelofibrosis with myeloid metaplasia; and Walden stroem disease; more suitably from rheumatoid arthritis (RA), pemphigus vulgaris (PV), idiopathic thrombocytopenia purpura (ITP), thrombotic thrombocytopenic purpura (TTP), autoimmune hemolytic anemia (AIHA), acquired hemophilia type A (AHA), systemic lupus erythematosus (SLE), multiple sclerosis (MS), myasthenia gravis (MG), Sjögren's syndrome (SS), ANCA-associated vasculitides, cryoglobulinemia, chronic autoimmune urticaria (CAU), allergy (atopic dermatitis, contact dermatitis, allergic rhinitis), goodpasture's syndrome, transplant rejection and cancers of haematopoietic origin as well as in disease or infection associated immunopathology, for example in severe and cerebral malaria, trypanosomiasis, leishmaniasis, toxoplasmosis and neurocysticercosis.

The pharmaceutical composition or combination of the present invention can be in unit dosage of about 1-1000 mg of active ingredient(s) for a subject of about 50-70 kg, or about 1-500 mg or about 1-250 mg or about 1-150 mg or about 0.5-100 mg, or about 1-50 mg of active ingredients. The therapeutically effective dosage of a compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, *e.g.*, mice, rats, dogs, monkeys or isolated organs, tissues and preparations thereof. A form of the present invention can be applied *in vitro* in the form of solutions, *e.g.*, aqueous solutions, and *in vivo* either enterally, parenterally, advantageously intravenously, *e.g.*, as a suspension or in aqueous solution. The dosage *in vitro* may range between about $10^{-3}$ molar and $10^{-9}$ molar concentrations. A therapeutically effective amount *in vivo* may range depending on the route of administration, between about 0.1-500 mg/kg, or between about 1-100 mg/kg.

A form of the present invention may be administered either simultaneously with, or before or after, one or more other therapeutic agent. A form of the present invention may be administered separately, by the same or different route of administration, or together in the same pharmaceutical composition as the other agents.

In one embodiment, the invention provides a product comprising an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and at least one other therapeutic agent as a combined preparation for simultaneous, separate or sequential use in therapy. In one embodiment, the therapy is the treatment of a disease or condition mediated by the activity of the PI3K enzymes. Products provided as a combined preparation include a composition comprising an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and the other therapeutic agent(s) together in the same pharmaceutical composition, or an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and the other therapeutic agent(s) in separate form, *e.g.* in the form of a kit.

In one embodiment, the invention provides a pharmaceutical composition comprising an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and another therapeutic agent(s). Optionally, the pharmaceutical composition may comprise a pharmaceutically acceptable carrier, as described above.

In one embodiment, the invention provides a kit comprising two or more separate pharmaceutical compositions, at least one of which contains an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone. In one embodiment, the kit comprises means for separately retaining said compositions, such as a container, divided bottle, or divided foil packet. An example of such a kit is a blister pack, as typically used for the packaging of tablets, capsules and the like.

The kit of the present invention may be used for administering different dosage forms, for example, oral and parenteral, for administering the separate compositions at different dosage intervals, or for titrating the separate compositions against one another. To assist compliance, the kit of the present invention typically comprises directions for administration.

In the combination therapies of the invention, the form of the invention and the other therapeutic agent may be manufactured and/or formulated by the same or different manufacturers. Moreover, the form of the invention and the other therapeutic may be brought together into a combination therapy: (i) prior to release of the combination product to physicians (*e.g.* in the case of a kit comprising the form of the invention and the other therapeutic agent); (ii) by the physician themselves (or under the guidance of the physician) shortly before administration; (iii) in the patient themselves, *e.g.* during sequential administration of the form of the invention and the other therapeutic agent.

The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use as a pharmaceutical.

Accordingly, the invention provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in treating a disease. The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in treating a disease or condition mediated by the activity of the PI3K enzymes, wherein the medicament is prepared for administration with another therapeutic agent. The invention also provides another therapeutic agent for use in treating a disease or condition mediated by the activity of the PI3K enzymes, wherein the medicament is administered with an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone.

The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in a method of treatment. The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in a method of treating a disease or condition mediated by the activity of the PI3K enzymes, wherein the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone is prepared for administration with another therapeutic agent. The invention also provides another therapeutic agent for use in a method of treating a disease or condition mediated by the activity of the PI3K enzymes, wherein the other therapeutic agent is prepared for administration with an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone. The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in a method of treating a disease or condition mediated by the activity of the PI3K enzymes wherein the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone is administered with another therapeutic agent. The invention also provides another therapeutic agent for use in a method of treating a disease or condition mediated by the activity of the PI3K enzymes wherein the other therapeutic agent is administered with an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone

The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in treating a disease. The invention also provides an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone for use in treating a disease or condition mediated by the activity of the PI3K enzymes, wherein the patient has previously (*e.g.* within 24 hours) been treated with another therapeutic agent. The invention also provides another therapeutic agent for use in treating a disease or condition mediated by the activity of the PI3K enzymes, wherein the patient has previously (*e.g.* within 24 hours) been treated with an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone.

The anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may be administered as the sole active ingredient or in conjunction with, e.g. as an adjuvant to, other drugs e.g. immunosuppressive or immunomodulating agents or other anti-inflammatory agents, e.g. for the treatment or prevention of allo- or xenograft acute or chronic rejection or inflammatory or autoimmune disorders, or a chemotherapeutic agent, e.g a malignant cell anti-proliferative agent. For example, the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may be used in combination with a calcineurin inhibitor, e.g. cyclosporin A or FK 506; a mTOR inhibitor, e.g. rapamycin, 40-O-(2-hydroxyethyl)-rapamycin, CCI779, ABT578, AP23573, TAFA-93, biolimus-7 or biolimus-9; an ascomycin having immuno-suppressive properties, e.g. ABT-281, ASM981, etc.; corticosteroids; cyclophosphamide; azathioprene; methotrexate; leflunomide; mizoribine; mycophenolic acid or salt; mycophenolate mofetil; 15-deoxyspergualine or an immunosuppressive homologue, analogue or derivative thereof; a PKC inhibitor, e.g. as disclosed in WO 02/38561 or WO 03/82859, e.g. the compound of Example 56 or 70; a JAK3 kinase inhibitor, e.g. N-benzyl-3,4-dihydroxy-benzylidene-cyanoacetamide $\alpha$-cyano-(3,4-dihydroxy)-]N-benzylcinnamamide (Tyrphostin AG 490), prodigiosin 25-C (PNU156804), [4-(4'-hydroxyphenyl)-amino-6,7-dimethoxyquinazoline] (WHI-P131), [4-(3'-bromo-4'-hydroxylphenyl)-amino-6,7-dimethoxyquinazoline] (WHI-P154), [4-(3',5'-dibromo-4'-hydroxylphenyl)-amino-6,7-dimethoxyquinazoline] WHI-P97, KRX-211, 3-{(3R,4R)-4-methyl-3-[methyl-(7H-pyrrolo[2,3-d]pyrimidin-4-yl)-amino]-piperidin-1-yl}-3-oxo-propionitrile, in free form or in a pharmaceutically acceptable salt form, e.g. mono-citrate (also called CP-690,550), or a compound as disclosed in WO 04/052359 or WO 05/066156; immunosuppressive monoclonal antibodies, e.g., monoclonal antibodies to leukocyte receptors, e.g., MHC, CD2, CD3, CD4, CD7, CD8, CD25, CD28, CD40, CD45, CD52, CD58, CD80, CD86 or their ligands; other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4Ig (for ex. designated ATCC 68629) or a mutant thereof, e.g. LEA29Y; adhesion molecule inhibitors, e.g. LFA-1 antagonists, ICAM-1 or -3 antagonists, VCAM-4 antagonists or VLA-4 antagonists; or antihistamines; or antitussives, or a bronchodilatory agent; or an angiotensin receptor blockers; or an anti-infectious agent.

Where the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone is administered in conjunction with other immunosuppressive / immunomodulatory, anti-inflammatory, chemotherapeutic or anti-infectious therapy, dosages of the co-administered immunosuppressant, immunomodulatory, anti-inflammatory, chemotherapeutic or anti-infectious compound will of course vary depending on the type of co-drug employed, e.g. whether it is a steroid or a calcineurin inhibitor, on the specific drug employed, on the condition being treated and so forth.

An anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may also be used to advantage in combination with other therapeutic agents, especially other antiproliferative agents. Such antiproliferative agents include, but are not limited to, aromatase inhibitors; antiestrogens; topoisomerase I inhibitors; topoisomerase II inhibitors; microtubule active agents; alkylating agents; histone deacetylase inhibitors; compounds, which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; agents used in the treatment of hematologic malignancies; compounds which target, decrease or inhibit the activity of

Flt-3; Hsp90 inhibitors; temozolomide (TEMODAL®); and leucovorin.

The term "aromatase inhibitor", as used herein, relates to a compound which inhibits the estrogen production, i.e., the conversion of the substrates androstenedione and testosterone to estrone and estradiol, respectively. The term includes, but is not limited to, steroids, especially atamestane, exemestane and formestane; and, in particular, non-steroids, especially aminoglutethimide, roglethimide, pyridoglutethimide, trilostane, testolactone, ketoconazole, vorozole, fadrozole, anastrozole and letrozole. Exemestane can be administered, e.g., in the form as it is marketed, e.g., under the trademark AROMASIN. Formestane can be administered, e.g., in the form as it is marketed, e.g., under the trademark LENTARON. Fadrozole can be administered, e.g., in the form as it is marketed, e.g., under the trademark AFEMA. Anastrozole can be administered, e.g., in the form as it is marketed, e.g., under the trademark ARIMIDEX. Letrozole can be administered, e.g., in the form as it is marketed, e.g., under the trademark FEMARA or FEMAR. Aminoglutethimide can be administered, e.g., in the form as it is marketed, e.g., under the trademark ORIMETEN. A combination of the invention comprising a chemotherapeutic agent which is an aromatase inhibitor is particularly useful for the treatment of hormone receptor positive tumors, e.g., breast tumors.

The term "anti-estrogen", as used herein, relates to a compound which antagonizes the effect of estrogens at the estrogen receptor level. The term includes, but is not limited to, tamoxifen, fulvestrant, raloxifene and raloxifene hydrochloride. Tamoxifen can be administered, e.g., in the form as it is marketed, e.g., under the trademark NOLVADEX. Raloxifene hydrochloride can be administered, e.g., in the form as it is marketed, e.g., under the trademark EVISTA. Fulvestrant can be formulated as disclosed in U.S. Patent No. 4,659,516 or it can be administered, e.g., in the form as it is marketed, e.g., under the trademark FASLODEX. A combination of the invention comprising a chemotherapeutic agent which is an antiestrogen is particularly useful for the treatment of estrogen receptor positive tumors, e.g., breast tumors.

The term "anti-androgen", as used herein, relates to any substance which is capable of inhibiting the biological effects of androgenic hormones and includes, but is not limited to, bicalutamide (CASODEX), which can be formulated, e.g., as disclosed in U.S. Patent No. 4,636,505.

The term "gonadorelin agonist", as used herein, includes, but is not limited to, abarelix, goserelin and goserelin acetate. Goserelin is disclosed in U.S. Patent No. 4,100,274 and can be administered, e.g., in the form as it is marketed, e.g., under the trademark ZOLADEX. Abarelix can be formulated, e.g., as disclosed in U.S. Patent No. 5,843,901. The term "topoisomerase I inhibitor", as used herein, includes, but is not limited to, topotecan, gimatecan, irinotecan, camptothecian and its analogues, 9-nitrocamptothecin and the macromolecular camptothecin conjugate PNU-166148 (compound A1 in WO 99/17804). Irinotecan can be administered, e.g., in the form as it is marketed, e.g., under the trademark CAMPTOSAR. Topotecan can be administered, e.g., in the form as it is marketed, e.g., under the trademark HYCAMTIN.

The term "topoisomerase II inhibitor", as used herein, includes, but is not limited to, the anthracyclines, such as doxorubicin, including liposomal formulation, e.g., CAELYX; daunorubicin; epirubicin; idarubicin; nemorubicin; the anthraquinones mitoxantrone and losoxantrone; and the podophillotoxines etoposide and teniposide. Etoposide can be administered, e.g., in the form as it is marketed, e.g., under the trademark ETOPOPHOS. Teniposide can be administered, e.g., in the form as it is marketed, e.g., under the trademark VM 26-BRISTOL. Doxorubicin can be administered, e.g., in the form as it is marketed, e.g., under the trademark ADRIBLASTIN or ADRIAMYCIN. Epirubicin can be administered, e.g., in the form as it is marketed, e.g., under the trademark FARMORUBICIN. Idarubicin can be administered, e.g., in the form as it is marketed, e.g., under the trademark ZAVEDOS. Mitoxantrone can be administered, e.g., in the form as it is marketed, e.g., under the trademark NOVANTRON.

The term "microtubule active agent" relates to microtubule stabilizing, microtubule destabilizing agents and microtublin polymerization inhibitors including, but not limited to, taxanes, e.g., paclitaxel and docetaxel; vinca alkaloids, e.g., vinblastine, especially vinblastine sulfate; vincristine, especially vincristine sulfate and vinorelbine; discodermolides; cochicine; and epothilones and derivatives thereof, e.g., epothilone B or D or derivatives thereof. Paclitaxel may be administered, e.g., in the form as it is marketed, e.g., TAXOL. Docetaxel can be administered, e.g., in the form as it is marketed, e.g., under the trademark TAXOTERE. Vinblastine sulfate can be administered, e.g., in the form as it is marketed, e.g., under the trademark VINBLASTIN R.P. Vincristine sulfate can be administered, e.g., in the form as it is marketed, e.g., under the trademark FARMISTIN. Discodermolide can be obtained, e.g., as disclosed in U.S. Patent No. 5,010,099. Also included are epothilone derivatives which are disclosed in WO 98/10121, U.S. Patent No. 6,194,181, WO 98/25929, WO 98/08849, WO 99/43653, WO 98/22461 and WO 00/31247. Especially preferred are epothilone A and/or B.

The term "alkylating agent", as used herein, includes, but is not limited to, cyclophosphamide, ifosfamide, melphalan or nitrosourea (BCNU or Gliadel). Cyclophosphamide can be administered, e.g., in the form as it is marketed, e.g., under the trademark CYCLOSTIN. Ifosfamide can be administered, e.g., in the form as it is marketed, e.g., under the trademark HOLOXAN.

The term "histone deacetylase inhibitors" or "HDAC inhibitors" relates to compounds which inhibit the histone deacetylase and which possess antiproliferative activity. This includes compounds disclosed in WO 02/22577, especially *N*-hydroxy-3-[4-[[(2-hydroxyethyl)[2-(1*H*-indol-3-yl)ethyl]-amino]methyl]phenyl]-2*E*-2-propenamide, *N*-hydroxy-3-[4-[[[2-(2-methyl-1 H-indol-3-yl)-ethyl]-amino]methyl]phenyl]-2E-2-propenamide and pharmaceutically acceptable salts thereof. It further especially includes suberoylanilide hydroxamic acid (SAHA).

The term "antineoplastic antimetabolite" includes, but is not limited to, 5-fluorouracil or 5-FU; capecitabine; gemcitabine; DNA demethylating agents, such as 5-azacytidine and decitabine; methotrexate and edatrexate; and folic acid antagonists, such as pemetrexed. Capecitabine can be administered, e.g., in the form as it is marketed, e.g., under the trademark XELODA. Gemcitabine can be administered, e.g., in the form as it is marketed, e.g., under the trademark GEMZAR. Also included is the monoclonal antibody trastuzumab which can be administered, e.g., in the form as it is marketed, e.g., under the trademark HERCEPTIN.
The term "platin compound", as used herein, includes, but is not limited to, carboplatin, *cis*-platin, cisplatinum and oxaliplatin. Carboplatin can be administered, e.g., in the form as it is marketed, e.g., under the trademark CARBOPLAT. Oxaliplatin can be administered, e.g., in the form as it is marketed, e.g., under the trademark ELOXATIN. The term "compounds targeting/decreasing a protein or lipid kinase activity; or a protein or lipid phosphatase activity; or further anti-angiogenic compounds", as used herein, includes, but is not limited to, protein tyrosine kinase and/or serine and/or threonine kinase inhibitors or lipid kinase inhibitors, e.g.,

a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g., a *N*-phenyl-2-pyrimidine-amine derivative, e.g., imatinib, SU101, SU6668 and GFB-111;

b) compounds targeting, decreasing or inhibiting the activity of the fibroblast growth factor-receptors (FGFR);

c) compounds targeting, decreasing or inhibiting the activity of the insulin-like growth factor receptor I (IGF-IR), such as compounds which target, decrease or inhibit the activity of IGF-IR, especially compounds which inhibit the IGF-IR receptor, such as those compounds disclosed in WO 02/092599;

d) compounds targeting, decreasing or inhibiting the activity of the Trk receptor tyrosine kinase family;

e) compounds targeting, decreasing or inhibiting the activity of the Axl receptor tyrosine kinase family;

f) compounds targeting, decreasing or inhibiting the activity of the c-Met receptor;

g) compounds targeting, decreasing or inhibiting the activity of the Kit/SCFR receptor tyrosine kinase;

h) compounds targeting, decreasing or inhibiting the activity of the C-kit receptor tyrosine kinases - (part of the PDGFR family), such as compounds which target, decrease or inhibit the activity of the c-Kit receptor tyrosine kinase family, especially compounds which inhibit the c-Kit receptor, e.g., imatinib;

i) compounds targeting, decreasing or inhibiting the activity of members of the c-Abl family and their gene-fusion products, e.g., BCR-Abl kinase, such as compounds which target decrease or inhibit the activity of c-Abl family members and their gene fusion products, e.g., a *N*-phenyl-2-pyrimidine-amine derivative, e.g., imatinib, PD180970, AG957, NSC 680410 or PD173955 from ParkeDavis;

j) compounds targeting, decreasing or inhibiting the activity of members of the protein kinase C (PKC) and Raf family of serine/threonine kinases, members of the MEK, SRC, JAK, FAK, PDK and Ras/MAPK family members, or PI(3) kinase family, or of the PI(3)-kinase-related kinase family, and/or members of the cyclin-dependent kinase family (CDK) and are especially those staurosporine derivatives disclosed in U.S. Patent No. 5,093,330, e.g., midostaurin; examples of further compounds include, e.g., UCN-01; safingol; BAY 43-9006; Bryostatin 1; Perifosine; Ilmofosine; RO 318220 and RO 320432; GO 6976; Isis 3521; LY333531/LY379196; isochinoline compounds, such as those disclosed in WO 00/09495; FTIs; PD184352; or QAN697 (a P13K inhibitor);

k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810, AG 99, Tyrphostin AG 213, Tyrphostin AG 1748, Tyrphostin AG 490, Tyrphostin B44, Tyrphostin B44 (+) enantiomer, Tyrphostin AG 555, AG 494, Tyrphostin AG 556, AG957 and adaphostin (4-{[(2,5-dihydroxyphenyl)methyl]amino}-benzoic acid adamantyl ester, NSC 680410, adaphostin; and

l) compounds targeting, decreasing or inhibiting the activity of the epidermal growth factor family of receptor tyrosine

kinases (EGFR, ErbB2, ErbB3, ErbB4 as homo- or hetero-dimers), such as compounds which target, decrease or inhibit the activity of the epidermal growth factor receptor family are especially compounds, proteins or antibodies which inhibit members of the EGF receptor tyrosine kinase family, e.g., EGF receptor, ErbB2, ErbB3 and ErbB4 or bind to EGF or EGF related ligands, and are in particular those compounds, proteins or monoclonal antibodies generically and specifically disclosed in WO 97/02266, e.g., the compound of Example 39, or in EP 0 564 409; WO 99/03854; EP 0520722; EP 0 566 226; EP 0 787 722; EP 0 837 063; U.S. Patent No. 5,747,498; WO 98/10767; WO 97/30034; WO 97/49688; WO 97/38983 and, especially, WO 96/30347, e.g., compound known as CP 358774; WO 96/33980, e.g., compound ZD 1839; and WO 95/03283, e.g., compound ZM105180, e.g., trastuzumab (HERCEPTIN), cetuximab, Iressa, Tarceva, OSI-774, CI-1033, EKB-569, GW-2016, E1.1, E2.4, E2.5, E6.2, E6.4, E2.11, E6.3 or E7.6.3; and

7*H*-pyrrolo-[2,3-*d*]pyrimidine derivatives which are disclosed in WO 03/013541. Further anti-angiogenic compounds include compounds having another mechanism for their activity, e.g., unrelated to protein or lipid kinase inhibition, e.g., thalidomide (THALOMID) and TNP-470.
Compounds which target, decrease or inhibit the activity of a protein or lipid phosphatase are, e.g., inhibitors of phosphatase 1, phosphatase 2A, PTEN or CDC25, e.g., okadaic acid or a derivative thereof.
Compounds which induce cell differentiation processes are e.g. retinoic acid, α- γ- or δ-tocopherol or α- γ- or δ-tocotrienol.
The term cyclooxygenase inhibitor, as used herein, includes, but is not limited to, e.g., Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g., 5-methyl-2-(2'-chloro-6'-fluoroanilino)phenyl acetic acid or lumiracoxib.
The term "bisphosphonates", as used herein, includes, but is not limited to, etridonic, clodronic, tiludronic, pamidronic, alendronic, ibandronic, risedronic and zoledronic acid. "Etridonic acid" can be administered, e.g., in the form as it is marketed, e.g., under the trademark DIDRONEL. "Clodronic acid" can be administered, e.g., in the form as it is marketed, e.g., under the trademark BONEFOS. "Tiludronic acid" can be administered, e.g., in the form as it is marketed, e.g., under the trademark SKELID. "Pamidronic acid" can be administered, e.g., in the form as it is marketed, e.g., under the trademark AREDIA™. "Alendronic acid" can be administered, e.g., in the form as it is marketed, e.g., under the trademark FOSAMAX. "Ibandronic acid" can be administered, e.g., in the form as it is marketed, e.g., under the trademark BONDRANAT. "Risedronic acid" can be administered, e.g., in the form as it is marketed, e.g., under the trademark ACTONEL. "Zoledronic acid" can be administered, e.g., in the form as it is marketed, e.g., under the trademark ZOMETA.
The term "mTOR inhibitors" relates to compounds which inhibit the mammalian target of rapamycin (mTOR) and which possess antiproliferative activity, such as sirolimus (Rapamune®), everolimus (Certican™), CCI-779 and ABT578.

The term "heparanase inhibitor", as used herein, refers to compounds which target, decrease or inhibit heparin sulphate degradation. The term includes, but is not limited to, PI-88.
The term "biological response modifier", as used herein, refers to a lymphokine or interferons, e.g., interferon γ.
The term "inhibitor of Ras oncogenic isoforms", e.g., H-Ras, K-Ras or N-Ras, as used herein, refers to compounds which target, decrease or inhibit the oncogenic activity of Ras, e.g., a "farnesyl transferase inhibitor", e.g., L-744832, DK8G557 or R115777 (Zarnestra).
The term "telomerase inhibitor", as used herein, refers to compounds which target, decrease or inhibit the activity of telomerase. Compounds which target, decrease or inhibit the activity of telomerase are especially compounds which inhibit the telomerase receptor, e.g., telomestatin.
The term "methionine aminopeptidase inhibitor", as used herein, refers to compounds which target, decrease or inhibit the activity of methionine aminopeptidase. Compounds which target, decrease or inhibit the activity of methionine aminopeptidase are, e.g., bengamide or a derivative thereof.
The term "proteasome inhibitor", as used herein, refers to compounds which target, decrease or inhibit the activity of the proteasome. Compounds which target, decrease or inhibit the activity of the proteasome include, e.g., PS-341 and MLN 341.
The term "matrix metalloproteinase inhibitor" or "MMP inhibitor", as used herein, includes, but is not limited to, collagen peptidomimetic and nonpeptidomimetic inhibitors, tetracycline derivatives, e.g., hydroxamate peptidomimetic inhibitor batimastat and its orally bioavailable analogue marimastat (BB-2516), prinomastat (AG3340), metastat (NSC 683551) BMS-279251, BAY 12-9566, TAA211, MMI270B or AAJ996.
The term "agents used in the treatment of hematologic malignancies", as used herein, includes, but is not limited to, FMS-like tyrosine kinase inhibitors, e.g., compounds targeting, decreasing or inhibiting the activity of FMS-like tyrosine kinase receptors (Flt-3R); interferon, 1-b-*D*-arabinofuransylcytosine (ara-c) and bisulfan; and ALK inhibitors, e.g., compounds which target, decrease or inhibit anaplastic lymphoma kinase. Compounds which target, decrease or inhibit the activity of FMS-like tyrosine kinase receptors (Flt-3R) are especially compounds, proteins or antibodies which inhibit members of the Flt-3R receptor kinase family, e.g., PKC412, midostaurin, a staurosporine derivative, SU11248 and MLN518.
The term "HSP90 inhibitors", as used herein, includes, but is not limited to, compounds targeting, decreasing or inhibiting

the intrinsic ATPase activity of HSP90; degrading, targeting, decreasing or inhibiting the HSP90 client proteins via the ubiquitin proteasome pathway. Compounds targeting, decreasing or inhibiting the intrinsic ATPase activity of HSP90 are especially compounds, proteins or antibodies which inhibit the ATPase activity of HSP90, e.g., 17-allylamino,17-demethoxygeldanamycin (17AAG), a geldanamycin derivative, other geldanamycin related compounds, radicicol and HDAC inhibitors.

The term "antiproliferative antibodies", as used herein, includes, but is not limited to, trastuzumab (Herceptin™), Trastuzumab-DM1, erlotinib (Tarceva™), bevacizumab (Avastin™), rituximab (Rituxan®), PRO64553 (anti-CD40) and 2C4 antibody. By antibodies is meant, e.g., intact monoclonal antibodies, polyclonal antibodies, multispecific antibodies formed from at least two intact antibodies, and antibodies fragments so long as they exhibit the desired biological activity.

For the treatment of acute myeloid leukemia (AML), the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone can be used in combination with standard leukemia therapies, especially in combination with therapies used for the treatment of AML. In particular, the anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone can be administered in combination with, e.g., farnesyl transferase inhibitors and/or other drugs useful for the treatment of AML, such as Daunorubicin, Adriamycin, Ara-C, VP-16,
Teniposide, Mitoxantrone, Idarubicin, Carboplatinum and PKC412.

An anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may also be used to advantage combination with other therapeutic agents, especially other anti-malarial agents. Such anti-malarial agents include, but are not limited to proguanil, chlorproguanil, trimethoprim, chloroquine, mefloquine, lumefantrine, atovaquone, pyrimethamine-sulfadoxine, pyrimethamine-dapsone, halofantrine, quinine, quinidine, amodiaquine, amopyroquine, sulphonamides, artemisinin, arteflene, artemether, artesunate, primaquine, inhaled NO, L-arginine, Dipropylenetri-amine NONOate (NO donor), Rosiglitzone (PPARγ agonist), activated charcoal, Erythropoietin, Levamisole, and pyronaridine.

An anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may also be used to advantage in combination with other therapeutic agents, such as used for the treatment of Leishmaniosis, Trypanosomiasis, Toxoplasmosis and Neurocysticercosis. Such agents include, but are not limited to chloroquine sulfate, atovaquone-proguanil, artemether-lumefantrine, quinine-sulfate, artesunate, quinine, doxycycline, clindamycin, meglumine antimoniate, sodium stibogluconate, miltefosine, ketoconazole, pentamidine, amphotericin B (AmB), liposomal-AmB, paromomycine, eflornithine, nifurtimox, suramin, melarsoprol, prednisolone, benznidazole, sulfadiazine, pyrimethamine, clindamycin, trimetropim, sulfamethoxazole, azitromycin, atovaquone, dexamethasone, praziquantel, albendazole, beta-lactams, fluoroquinolones, macrolides, aminoglycosides, sulfadiazine and pyrimethamine.

The structure of the active agents identified by code nos., generic or trade names may be taken from the actual edition of the standard compendium "The Merck Index" or from databases, e.g., Patents International, e.g., IMS World Publications.

The above-mentioned compounds, which can be used in combination with an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone, can be prepared and administered as described in the art, such as in the documents cited above.

An anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may also be used to advantage in combination with known therapeutic processes, e.g., the administration of hormones or especially radiation.

An anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone may in particular be used as a radiosensitizer, especially for the treatment of tumors which exhibit poor sensitivity to radiotherapy.

By "combination", there is meant either a fixed combination in one dosage unit form, or a kit of parts for the combined administration where an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and a combination partner may be administered independently at the same time or separately within time intervals that especially allow that the combination partners show a cooperative, e.g., synergistic, effect or any combination thereof. The terms "coadministration" or "combined administration" or the like as utilized herein are meant to encompass administration of the selected combination partner to a single subject in need thereof (e.g. a patient), and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g. an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone and a combination partner, are both administered to a patient simultaneously in the form of a single entity or dosage. The term "non-fixed combination" means that the active ingredients, e.g. an anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone) and a combination partner, are both administered to a patient as separate entities either simultaneously, concurrently or sequentially with no specific time limits, wherein such administration provides therapeutically effective levels of the two compounds in the body of the patient. The latter also applies to cocktail therapy, e.g. the administration of three or more active ingredients.

EXAMPLES

**Experimental details:**

The following examples are intended to illustrate the invention and are not to be construed as being limitations thereon. Temperatures are given in degrees Celsius. If not mentioned otherwise, all evaporations are performed under reduced pressure, typically between about 15 mm Hg and 100 mm Hg (= 20-133 mbar). The structure of final products, intermediates and starting materials is confirmed by standard analytical methods, e.g., microanalysis and spectroscopic characteristics, e.g., MS, IR, NMR. Abbreviations used are those conventional in the art.

All starting materials, building blocks, reagents, acids, bases, dehydrating agents, solvents, and catalysts utilized to synthesis the forms of the present invention are either commercially available or can be produced by organic synthesis methods known to one of ordinary skill in the art (Houben-Weyl 4th Ed. 1952, Methods of Organic Synthesis, Thieme, Volume 21). Further, the forms of the present invention can be produced by organic synthesis methods known to one of ordinary skill in the art as shown in the following examples.

Abbreviations

| | |
|---|---|
| ACN | acetonitrile |
| AcOH | acetic acid |
| aq. | aqueous |
| Boc | *tert*-butoxycarbonyl |
| $Boc_2O$ | di-*tert*-butyl dicarbonate |
| tBu | *tert*-butyl |
| tBuOH | *tert*-butanol |
| BrettPhos | 2-(Dicyclohexylphosphino)-3,6-dimethoxy-2'-4'-6'-triisopropyl-1,1'-biphenyl |
| br s | broad singlet |
| COMU | (1-cyano-2-ethoxy-2-oxoethylidenaminooxy)dimethylamino-morpholino-carbenium hexafluorophosphate |
| conc. | concentrated |
| d | day(s) |
| d | doublet |
| dd | doublet of doublets |
| dba | dibenzylideneacetone |
| DCM | dichloromethane |
| DEA | diethylamine |
| DEAD | diethyl azodicarboxylate |
| DEAP | diethylaminopyridine |
| DIPEA | diisopropylethylamine |
| DMF | dimethylformamide |
| DMME | dimethoxymethane |
| DMSO | dimethylsulfoxide |
| DPPA | diphenylphosphoryl azide |
| DPPF | 1,1'-bis(diphenylphosphino)ferrocene |
| EDC | 1-(3-dimethylaminopropyl)-3-ethylcarbodiimide hydrochloride |
| eq. | equivalent(s) |
| ESI | electrospray ionisation |
| $Et_3N$ | triethylamine |
| $Et_2O$ | diethylether |

| | |
|---|---|
| EtOAc | ethyl acetate |
| EtOH | ethanol |
| h | hour(s) |
| HATU | *O*-(7-azabenzotriazol-1-yl)-*N,N,N',N'*-tetramethyluronium hexafluorophosphate |
| HBTU | O-(1H-benzotriazol-1-yl)-*N,N,N',N'*-tetramethyluronium hexafluorophosphate |
| HMDS | hexamethyldisilazane |
| HOBT | 1-hydroxy-benztriazole |
| HPLC | high performance liquid chromatography |
| I PA | isopropanol |
| LCMS | liquid chromatography with mass spectrometry |
| mCPBA | meta-chloroperoxybenzoic acid |
| MeOH | methanol |
| m | multiplet |
| min | minute(s) |
| MS | mass spectrometry |
| mw | microwave |
| NMR | nuclear magnetic resonance spectrometry |
| NaOtBu | sodium tert-butoxide |
| NP | normal phase |
| OBD | optimum bed density |
| $Pd_2(dba)_3$ | tris(dibenzylideneacetone)dipalladium |
| PL-$HCO_3$ MP SPE | Polymer-supported bicarbonate cartridge for acid removal |
| prep. | preparative |
| $PPh_3$ | triphenylphosphine |
| q | quartet |
| Rac-BINAP | racemic 2,2'-bis(di-*p*-tolylphosphino)-1,1'-binaphthyl |
| RP | reversed phase |
| Rt | retention time |
| rt | room temperature |
| RuPhos | 2-dicyclohexylphosphino-2',6'-di-isopropoxy-1,1'-biphenyl |
| sat. | saturated |
| SCX-2 | polymer supported sulfonic acid macroporous polystyrene |
| soln. | solution |
| t | triplet |
| TBME | *tert*-butyl methyl ether |
| TBAF | tetrabutylammonium fluoride |
| TBDMSCI | *tert*-butyldimethylsilylchloride |
| Tetramethyl-t-butyl -XPhos | 2-di-t-butylphosphino-3,4,5,6-tetramethyl-2',4',6'-triisopropylbiphenyl |
| TFA | trifluoroacetic acid |
| THF | tetrahydrofuran |
| TLC | thin layer chromatography |
| UPLC | ultra performance liquid chromatography |
| XPhos | 2-dicyclohexylphosphino-2',4',6'-triisopropylbiphenyl |
| Pd[RuPhos] | (2-dicyclohexylphosphino-2' 6'-diisopropyl-1 1'-biphenyl)(2-(2-aminoethyl)phenyl)palladium(II) |

Microwave equipment used is a Biotage Initiator®

All compounds are named using AutoNom.

<u>General chromatography information</u>

**LCMS method M1 ($Rt_{M1}$)**

| | |
|---|---|
| HPLC-column dimensions: | 2.1 x 50 mm |
| HPLC-column type: | Acquity UPLC HSS T3, 1.8 μm |

(continued)

| | |
|---|---|
| HPLC-eluent: | A) water + 0.05 Vol.-% formic acid + 3.75 mM ammonium acetate B) ACN + 0.04 Vol.-% formic acid |
| HPLC-gradient: | 2 - 98% B in 1.4 min, 98% B 0.45 min, flow = 1.2 ml / min |
| HPLC-column temperature: | 50 °C |

**LCMS method M2 ($Rt_{M2}$)**

| | |
|---|---|
| HPLC-column dimensions: | 2.1 x 30 mm |
| HPLC-column type: | Ascentis Express C18, 2.7 µm |
| HPLC-eluent | A) water + 0.05 Vol.-% formic acid + 3.75 mM ammonium acetate B) ACN + 0.04 Vol.-% formic acid |
| HPLC-gradient: | 2 - 98% B in 1.4 min, 0.75 min 98% B, flow = 1.2 ml / min |
| HPLC-column temperature: | 50 °C |

**LCMS method M3 ($Rt_{M3}$)**

| | |
|---|---|
| HPLC-column dimensions: | 2.1 x 30 mm |
| HPLC-column type: | Ascentis Express C18, 2.7 µm |
| HPLC-eluent | A) water + 0.05 Vol.-% formic acid + 3.75 mM ammonium acetate B) ACN + 0.04 Vol.-% formic acid |
| HPLC-gradient: | 2 - 98% B in 8.5 min, 1 min 98% B, flow = 1.2 ml / min |
| HPLC-column temperature: | 50 °C |

**LCMS method M4 ($Rt_{M4}$)**

| | |
|---|---|
| HPLC-column dimensions: | 4.6 x 50 mm |
| HPLC-column type: | SunFire C18, 5 µm |
| HPLC-eluent | A) water + 0.1 Vol.-% TFA, B) ACN + 0.1 Vol.-% TFA |
| HPLC-gradient: | 5 - 100% B in 8.0 min B, flow = 2 ml / min |
| HPLC-column temperature: | 40 °C |

**LCMS method M5 ($Rt_{M5}$)**

| | |
|---|---|
| HPLC-column dimensions: | 0.46x25 cm |
| HPLC-column type: | Chiralcel OJ-H (1189) |
| HPLC-eluent | EtOH/MeOH 60:40 |
| HPLC-gradient: | isocratic, flow=0.5ml/min |
| Detector: | UV 220 nm |

**LCMS method M6 ($Rt_{M6}$)**

| | |
|---|---|
| HPLC-column dimensions: | 2.1 x 30 mm |
| HPLC-column type: | Ascentis Express C18, 2.7 µm |
| HPLC-eluent | A) water + 0.05% TFA, B) ACN + 0.04% TFA |
| HPLC-gradient: | 2 - 98% B in 1.4 min, 0.75 min 98% B, flow = 1.2 ml / min |
| HPLC-column temperature: | 50 °C |

**LCMS method M7 ($Rt_{M7}$)**

| | |
|---|---|
| HPLC-column dimensions: | 2.1 x 30 mm |

(continued)

| | |
|---|---|
| HPLC-column type: | Ascentis Express C18, 2.7 μm |
| HPLC-eluent | A) water + 0.05% TFA, B) ACN + 0.04% TFA |
| HPLC-gradient: | 10 - 95% B in 3.0 min, 1 min 95% B, flow = 1.2 ml / min |
| HPLC-column temperature: | 50 °C |

**LCMS method M8 ($Rt_{M8}$)**

| | |
|---|---|
| HPLC-column dimensions: | 2.1 x 30 mm |
| HPLC-column type: | Ascentis Express C18 2.7 μm |
| HPLC-eluent: | A) water + 0.05% formic acid + 3.75 mM ammonium acetate, B) acetonitrile +0.04% formic acid |
| HPLC-gradient: | 10 - 95% B in 3.0 min, flow = 1.2 ml/min |

**LCMS method M9 ($Rt_{M9}$)**

| | |
|---|---|
| HPLC-column dimensions: | 2.1 x 30 mm |
| HPLC-column type: | Ascentis Express C18 2.7 μm |
| HPLC-eluent: | A) water + 0.05% formic acid + 3.75 mM ammonium acetate, B) acetonitrile +0.04% formic acid |
| HPLC-gradient: | 10% B from 0.0 to 0.5 min then from 0.5 min to 3.0 min gradient 10 - 95% B, flow = 1.2 ml/min |

**LCMS method M10 ($Rt_{M10}$)**

| | |
|---|---|
| HPLC-column dimensions: | 2.1 x 50 mm |
| HPLC-column type: | Acquity UPLC BEH C18 1.7 μm |
| HPLC-eluent: | A) water + 0.1 Vol.-% formic acid, B) acetonitrile |
| HPLC-gradient: | 20 - 25% B in 1.00 min, then 25 - 95% B in 3.20 min, then 95 - 100% B in 0.10 min, then 100% for 0.20 min, flow = 0.7 ml/min |

**LCMS method M11 ($Rt_{M11}$)**

| | |
|---|---|
| HPLC-column dimensions: | 2.1 x 50 mm |
| HPLC-column type: | Acquity UPLC BEH C18 1.7 μm |
| HPLC-eluent: | A) water + 0.1 Vol.-% formic acid, B) acetonitrile |
| HPLC-gradient: | 5 - 10% B in 1.00 min, then 10 - 90% B in 3.00 min, then 90 - 100% B in 0.10 min, then 100% for 0.40 min, flow = 0.7 ml/min |

**LCMS method M12 ($Rt_{M12}$)**

| | |
|---|---|
| HPLC-column dimensions: | 2.1 x 30 mm |
| HPLC-column type: | Ascentis Express C18 2.7 μm |
| HPLC-eluent: | A) water + 0.1 Vol.-% TFA, B) acetonitrile |
| H PLC-gradient: | 10 -95% B over 1.7 min and 1.2 mL/min as solvent flow and then 95 5 B over 0.7 min, flow = 1.4 mL/min. |

**LCMS method M13 ($Rt_{M13}$)**

| | |
|---|---|
| HPLC-column dimensions: | 2.1 x 30 mm |
| HPLC-column type: | Ascentis Express C18 2.7 μm |
| HPLC-eluent: | A) water + 0.05% formic acid + 3.75 mM ammonium acetate, |

(continued)

| | |
|---|---|
| | B) acetonitrile +0.04% formic acid |
| H PLC-gradient: | 10 - 95% B in 3.7 min, flow = 1.2 ml / min |

**LCMS method M14 ($Rt_{M14}$)**

| | |
|---|---|
| HPLC-column dimensions: | 2.1 x 30 mm |
| HPLC-column type: | Ascentis Express C18, 2.7 μm |
| HPLC-eluent | A) water + 0.05% formic acid + 3.75 mM ammonium acetate, B) acetonitrile +0.04% formic acid |
| H PLC-gradient: | 10 - 95% B in 1.5 min, 1 min 95% B, flow = 1.2 ml / min |

**LCMS method M15 ($Rt_{M15}$)**

| | |
|---|---|
| HPLC-column dimensions: | 0.46x25 cm |
| HPLC-column type: | Chiralcel OD-H (1194) |
| HPLC-eluent | Hexan/EtOH 50:50 + 0.05% DEA |
| H PLC-gradient: | isocratic, flow=0.5ml/min |
| Detector: | UV 220 nm |

**LCMS method M16 ($Rt_{M16}$)**

| | |
|---|---|
| HPLC-column dimensions: | 2.1 x 50 mm |
| HPLC-column type: | Acquity UPLC HSS T3, 1.8 μm |
| HPLC-eluent: | A) water + 0.05 Vol.-% formic acid + 3.75 mM ammonium acetate B) ACN + 0.04 Vol.-% formic acid |
| H PLC-gradient: | 5 - 98% B in 1.4 min, 98% B 0.4 min, flow = 1.0 ml / min |
| HPLC-column temperature: | 60 °C |

**X-ray Powder Diffraction**

**Instrumentation:**

**Method X1**

| | |
|---|---|
| Instrument | Bruker D8 GADDS Discover |
| Irradiation | CuKα (40 kV, 40 mA) |
| Detector | HI-STAR Area detector |
| Scan range | 6°-39° (2 theta value) |

**Melting Point determination:**

Melting point was determined by Differential Scanning calorimetry (DSC). DSC was as recorded on a TA Instruments DSC Q2000 using a heating rate of 10°C/min. A sample of 0.6 mg was weighed into standard aluminium pan (pan + lid, TA 900786.901, 900779.901). The instrument was operated using the Thermal Advantage Q-Series software V.2.6.0.367 and the Thermal Advantage software V4.6.9. Thermal events were characterized using Universal Analysis V4.3A Build 4.3.0.6. The samples was measured against sample pan without pin hole. The sample was treated according to the protocol below:

Step 1: EQUILIBRATE AT 0°C
Step 2: Ramp 10°C/min to 300°C

**Example F1: (1,1-Dioxo-hexahydro-1lambda*6*-thiopyran-4-yl)-{(S)-3-[1-(6-methoxy-5 -methyl-pyridin-3-yl)-2,3-dihydro-1H-pyrido[3,4-b][1,4]oxazin-7-yloxy]-pyrrolidin-1-yl}-methanone**

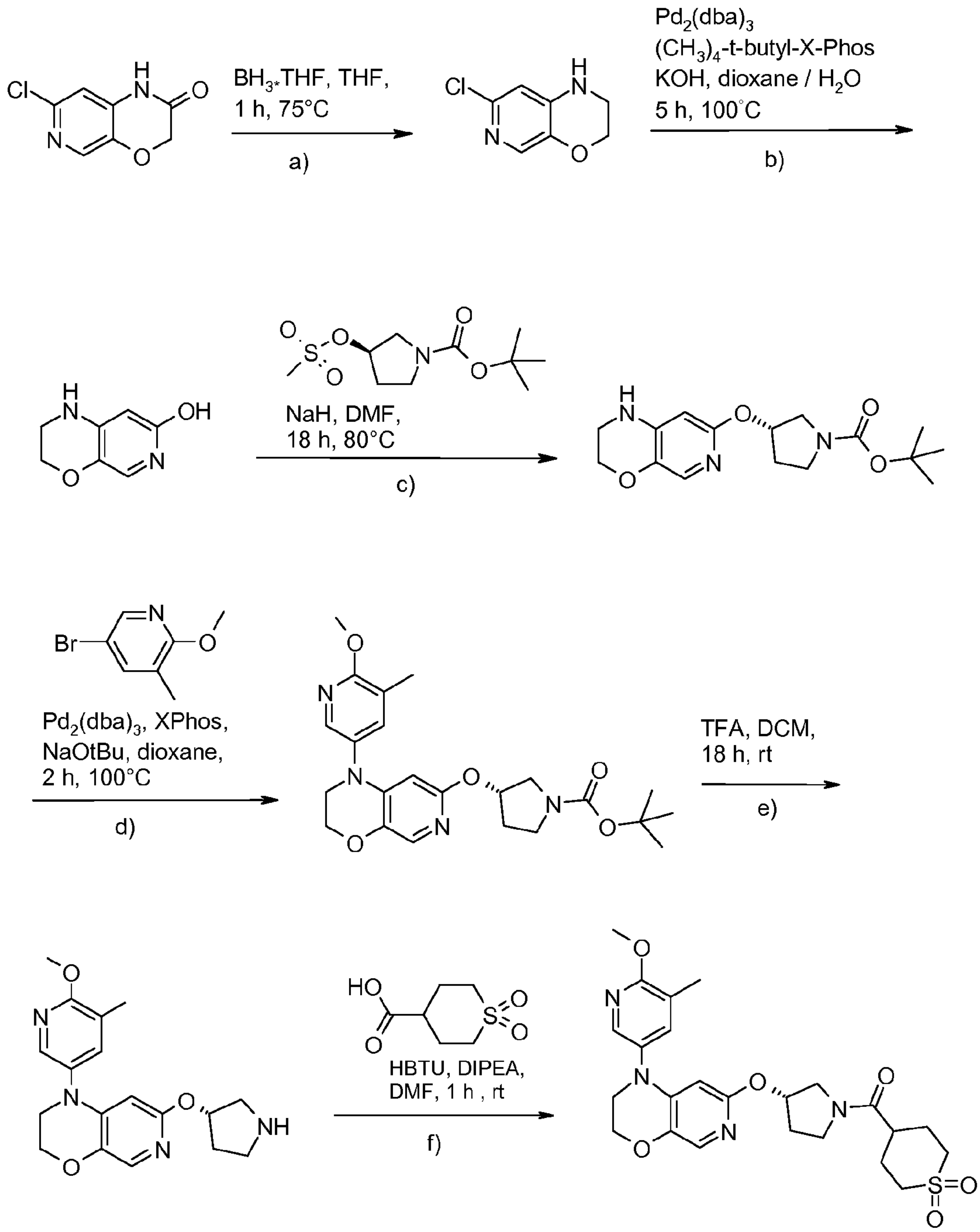


a) **7-Chloro-2,3-dihydro-1H-pyrido[3,4-b][1,4]oxazine**

A solution of 7-chloro-1H-pyrido[3,4-b][1,4]oxazin-2-one (CAS registry 928118-43-8) (3.70 g, 20 mmol) in THF (63 ml) was treated with $BH_3$*THF (1 M in THF, 47 ml, 47 mmol). The reaction mixture was stirred at 75°C for 1 h, then cooled down to rt and quenched with methanol (24 ml, 600 mmol). The reaction mixture was concentrated under reduced pressure and the residue was taken up with EtOAc and washed with sat. aq. $NaHCO_3$ soln. The organic layer was dried over $MgSO_4$, filtered and concentrated under reduced pressure to afford the title product as a pale yellow solid (3.3 g, 96% yield).

UPLC $Rt_{M1}$ =0.47 min; ESIMS: 171 [$(M+H)^+$].

$^1H$ NMR (400 MHz, DMSO-$d_6$): δ 7.53 (s, 1 H), 7.11 (br s, 1 H), 6.47 (s, 1 H), 4.09 (t, 2H), 3.17-3.38 (m, 2H).

**b) 2,3-Dihydro-1H-pyrido[3,4-b][1,4]oxazin-7-ol**

A mixture of 7-chloro-2,3-dihydro-1H-pyrido[3,4-b][1,4]oxazine (1.08 g, 6.33 mmol), aq. KOH soln. (1.07 g, 19 mmol KOH in 5.4 ml water), 2-di-t-butylphosphino-3,4,5,6-tetramethyl-2',4',6-tri-i-propylbiphenyl 98% (0.30 g, 0.63 mmol) and $Pd_2(dba)_3$ (0.29 g, 0.32 mmol) in dioxane (32.5 ml) was degassed three times with nitrogen, the tube was sealed and the reaction mixture was stirred at 100 °C for 5 h. After cooling to rt, the reaction mixture was filtered through hyflo, rinsed with EtOAc and methanol. The filtrates were concentrated and the title compound was obtained after flash chromatography on silica gel (DCM / MeOH, 98:2 to 75:25) as an orange residue (660 mg, 69% yield)
UPLC $Rt_{M1}$ =0.34 min; ESIMS: 153 [$(M+H)^+$].
$^1H$ NMR (400 MHz, DMSO-$d_6$): δ 10.33 (br s, 1 H), 7.03 (br s, 1 H), 6.71 (s, 1 H), 5.15 (s, 1 H), 3.95 (t, 2H), 3.25 (m, 2H).

**c) (S)-3-(2,3-Dihydro-1H-pyrido[3,4-b][1,4]oxazin-7-yloxy)-pyrrolidine-1-carboxylic acid tert-butyl ester**

A dry solution of 2,3-dihydro-1H-pyrido[3,4-b][1,4]oxazin-7-ol (0.66 g, 4.34 mmol) and (R)-3-methanesulfonyloxy-pyrrolidine-1-carboxylic acid tert-butyl ester (CAS registry 127423-61-4) (1.73 g, 6.51 mmol) in DMF (40 ml) was treated with sodium hydride (60% in mineral oil, 0.21 g, 8.68 mmol) and the reaction mixture was stirred at 80°C for 18 h. After cooling to rt, the reaction mixture was diluted with TBME and washed with sat. aq. $NaHCO_3$ soln.. The organic layer was dried over $MgSO_4$, filtered, concentrated and the title compound was obtained after flash chromatography on silica gel (cyclohexane / EtOAc, 95:5 to 30:70) as a yellow oil (1.035 g, 75% purity,, 56% yield)
UPLC $Rt_{M1}$ =0.65 min; ESIMS: 322 [$(M+H)^+$].
$^1H$ NMR (400 MHz, $CDCl_3$): δ 7.54 (s, 1 H), 5.86 (s, 1 H), 5.42 (br s, 1 H), 4.25-4.41 (m, 1 H), 4.19 (t, 2H), 3.38-3.66 (m, 6H), 2.00-2.18 (m, 2H), 1.46 (d, 9H).

**d) (S)-3-[1-(6-Methoxy-5-methyl-pyridin-3-yl)-2,3-dihydro-1H-pyrido[3,4-b][1,4]oxazin-7-yloxy]-pyrrolidine-1-carboxylic acid tert-butyl ester**

A mixture of (S)-3-(2,3-dihydro-1H-pyrido[3,4-b][1,4]oxazin-7-yloxy)-pyrrolidine-1-carboxylic acid tert-butyl ester (254 mg, 0.79 mmol), 5-bromo-2-methoxy-3-methylpyridine (CAS registry 760207-87-2) (208 mg, 1.03 mmol), XPhos (30 mg, 0.06 mmol), and NaOtBu (167 mg, 1.74 mmol) in dioxane (6 ml) was degassed with argon for 5 min, then $Pd_2(dba)_3$ (29 mg, 0.03 mmol) was added. The tube was filled with argon, sealed and the reaction mixture was stirred at 100°C for 2 h. After cooling to rt, the reaction mixture was filtered through hyflo, rinsed with EtOAc and the filtrates were washed with sat. aq. $NaHCO_3$ soln.. The aq. layer was twice reextracted with EtOAc, the combined organic layers were dried over $Na_2SO_4$, filtered, concentrated and the title compound was obtained after flash chromatography on silica gel (heptane / EtOAc, 100:0 to 50:50) as a clear gum (274 mg, 78% yield).UPLC $Rt_{M1}$ =1.20 min; ESIMS: 443 [$(M+H)^+$].
$^1H$ NMR (400 MHz, $CDCl_3$): δ 7.93 (d, 1 H), 7.61 (br s, 1 H), 7.30-7.35 (m, 1H), 5.71 (s, 1 H), 5.34-5.46 (m, 1 H), 4.31 (br s, 2H), 3.99 (s, 3H), 3.68 (t, 2H), 3.34-3.62 (m, 4H), 2.23 (s, 3H), 2.01-2.09 (m, 2H), 1.44 (s, 9H).

**e) 1-(6-Methoxy-5-methyl-pyridin-3-yl)-7-((S)-pyrrolidin-3-yloxy)-2,3-dihydro-1H-pyrido[3,4-b][1,4]oxazine**

A solution of (S)-3-[1-(6-methoxy-5-methyl-pyridin-3-yl)-2,3-dihydro-1H-pyrido[3,4-b][1,4]oxazin-7-yloxy]-pyrrolidine-1-carboxylic acid tert-butyl ester (364 mg, 0.82 mmol) in DCM (6 ml) was treated with TFA (0.63 ml, 8.23 mmol) and the reaction mixture was stirred at rt for 18 h, then quenched with sat. aq. $NaHCO_3$ soln. and extracted with DCM. The organic layer was dried over $MgSO_4$, filtered and concentrated under reduced pressure to afford the title product as a red oil, which was used in the next step without further purification (313mg, 90% purity, quantitative yield).
UPLC $Rt_{M1}$ =0.65 min; ESIMS: 343 [$(M+H)^+$].
$^1H$ NMR (400 MHz, $CDCl_3$): δ 7.93 (d, 1 H), 7.62 (s, 1 H), 7.32 (d, 1 H), 5.70 (s, 1 H), 5.26-5.36 (m, 1 H), 4.31 (t, 2H), 3.99 (s, 3H), 3.67 (t, 2H), 2.95-3.15 (m, 3H), 2.81-2.92 (m, 1 H), 2.22 (s, 3H), 1.98-2.10 (m, 1H), 1.79-1.90 (m, 1H).

**f) (1,1-Dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone**

A solution of 1,1-dioxo-hexahydro-1lambda*6*-thiopyran-4-carboxylic acid (CAS registry 64096-87-3) (106 mg, 0.59 mmol) in DMF (4 ml) was treated with HBTU (225 mg, 0.59 mmol) and DIPEA (0.24 ml, 1.37 mmol). The resulting orange solution was stirred at rt for 5 min, then a solution of 1-(6-methoxy-5-methyl-pyridin-3-yl)-7-((S)-pyrrolidin-3-yloxy)-2,3-dihydro-1H-pyrido[3,4-b][1,4]oxazine (156 mg, 0.46 mmol) in DMF (2 ml) was added. The reaction mixture was stirred at rt for 1 h then concentrated under reduced pressure and the residue was taken up with DCM and washed

with sat. aq. $NaHCO_3$ soln.. The organic layer was dried by passing it through a phase separating cartridge, concentrated and the title compound was obtained after SFC chromatography (column DEAP (250mm x 30mm, 60A, 5μm) Princeton, gradient 11 - 16% of methanol in supercritical $CO_2$ in 6 min) as a slightly coloured solid (112 mg, 49% yield).
UPLC $Rt_{M1}$ =0.81 min; ESIMS: 503 [(M+H)$^+$].
$^1$H NMR (400 MHz, DMSO-$d_6$): δ 8.01 (s, 1 H), 7.61 (m, 1 H), 7.52 (d, 1 H), 5.52 (d, 1 H), 5.24-5.43 (m, 1 H), 4.26 (br s, 2H), 3.89 (s, 3H), 3.59-3.79 (m, 3H), 3.41-3.56 (m, 2H), 3.21-3.39 (m, 1 H), 2.98-3.21 (m, 4H), 2.67-2.83 (m, 1 H), 1.84-2.20 (m, 9H).
$^1$H NMR (600 MHz, DMSO-$d_6$): δ 8.01 (s, 1 H), 7.63-7.59 (m, 1 H), 7.55-7.51 (m, 1 H), 5.55-5.51 (m, 1 H), 5.43-5.24 (m, 1 H), 4.29-4.22 (m, 2H), 3.90 (s, 3H), 3.80-3.60 (m, 2H), 3.56-3.37 (m, 3H), 3.28-2.99 (m, 5H), 2.89-2.66 (m, 1 H), 2.19-2.09 (m, 4H), 2.08-1.98 (m, 2H), 1.98-1.86 (m, 3H).

**Crystallization of Example F1 by heating and cooling in isopropanol / diethyl ether**

474mg of amorphous Example F1 was suspended in 1.4mL of isopropanol. The mixture was heated to 70°C and stirred at 70°C to allow complete dissolution of Example F1. The solution was cooled down to RT, a glue residue was formed. 2mL of diethyl ether was added and the slurry was stirred for 48h. A white suspension was formed. The suspension was filtered and the solid was dried at 40°C, 15mbar. A fine, white powder was obtained. The material contains only slight residual solvent (<0.5%). A crystalline anhydrous form of Example F1 with an onset melting of 148.77°C was obtained.

List of most significant 2-Theta peaks from X-ray Powder Diffraction Pattern with tolerances ±0.5 of Example F1 anhydrous form (Method M1) (including low/weak peaks for information). Note: This list of peaks is not exhaustive but are only "inter alia".

| 2-Theta in deg | Intensity |
|---:|---:|
| 9.1 | Low |
| 10.2 | Medium |
| 11.9 | Medium |
| 13.0 | Low |
| 17.1 | Strong |
| 17.7 | Medium, unresolved |
| 18.7 | Medium |
| 20.3 | Medium, unresolved |
| 20.8 | Medium, unresolved |
| 26.0 | Medium/low |
| 26.7 | Medium |
| 23.2 | Medium/low |
| 24.1 | Medium/low |
| 24.8 | Medium/low |
| 29.3 | Medium/low |
| 27.4 | Medium/low |
| 21.4 | Medium/low |

__Biological evaluation__

The activity of a compound can be assessed by the following *in vitro* & *in vivo* methods.

## Biological assays

### 1 Determination of enzymatic PI3K alpha and PI3K delta isoform inhibition

#### 1.1 Test of lipid kinase activity

The efficacy of the compounds as PI3 kinase inhibitors can be demonstrated as follows:

The kinase reaction is performed in a final volume of 50 μl per well of a half area COSTAR, 96 well plate. The final concentrations of ATP and phosphatidyl inositol in the assay are 5 μM and 6 μg/mL, respectively. The reaction is started by the addition of PI3 kinase, e.g. PI3 kinase δ.

**p110δ.** The components of the assay are added per well as follows:

- 10 μl test compound in 5% DMSO per well in columns 2-1.
- Total activity is determined by addition 10 μl of 5% vol/vol DMSO in the first 4 wells of column 1 and the last 4 wells of column 12.
- The background is determined by addition of 10 μM control compound to the last 4 wells of column 1 and the first 4 wells of column 12.
- 2 mL 'Assay mix' are prepared per plate:

  1.912 mL of HEPES assay buffer

  8.33 μl of 3 mM stock of ATP giving a final concentration of 5 μM per well

  1 μl of [$^{33}$P]ATP on the activity date giving 0.05 μCi per well

  30 μl of 1 mg/mL PI stock giving a final concentration of 6 μg/mL per well

  5 μl of 1 M stock $MgCl_2$ giving a final concentration of 1 mM per well

- 20 μl of the assay mix are added per well.
- 2 mL 'Enzyme mix' are prepared per plate (x* μl PI3 kinase p110β in 2 mL of kinase buffer). The 'Enzyme mix' is kept on ice during addition to the assay plates.
- 20 μl 'Enzyme mix' are added/well to start the reaction.
- The plate is then incubated at room temperature for 90 minutes.
- The reaction is terminated by the addition of 50 μl WGA-SPA bead (wheat germ agglutinin-coated Scintillation Proximity Assay beads) suspension per well.
- The assay plate is sealed using TopSeal-S (heat seal for polystyrene microplates, PerkinElmer LAS [Deutschland] GmbH, Rodgau, Germany) and incubated at room temperature for at least 60 minutes.
- The assay plate is then centrifuged at 1500 rpm for 2 minutes using the Jouan bench top centrifuge (Jouan Inc., Nantes, France).
- The assay plate is counted using a Packard TopCount, each well being counted for 20 seconds.

  * The volume of enzyme is dependent on the enzymatic activity of the batch in use.

In a more preferred assay, the kinase reaction is performed in a final volume of 10 μl per well of a low volume non-binding CORNING, 384 well black plate (Cat. No. #3676). The final concentrations of ATP and phosphatidyl inositol (PI) in the assay are 1 μM and 10 μg/mL, respectively. The reaction is started by the addition of ATP.

The components of the assay are added per well as follows:

50 nl test compounds in 90% DMSO per well, in columns 1-20, 8 concentrations (1/3 and 1/3.33 serial dilution step) in single.

- Low control: 50 nl of 90% DMSO in half the wells of columns 23-24 (0.45% in final).
- High control: 50 nl of reference compound (e.g. compound of Example 7 in WO 2006/122806) in the other half of columns 23-24 (2.5 μM in final).
- Standard: 50 nl of reference compound as just mentioned diluted as the test compounds in columns 21-22.
- 20 mL 'buffer' are prepared per assay :

  200 μl of 1M TRIS HCl pH7.5 (10 mM in final)
  60 μl of 1 M $MgCl_2$ (3 mM in final)
  500 μl of 2M NaCl (50 mM in final)
  100 μl of 10% CHAPS (0.05% in final)
  200 μl of 100mM DTT (1 mM in final)
  18.94 mL of nanopure water

- 10 mL 'PI' are prepared per assay :

  200 μl of 1 mg/mL I-alpha-Phosphatidylinositol (Liver Bovine, Avanti Polar Lipids Cat. No. 840042C MW=909.12) prepared in 3% OctylGlucoside (10 μg/mL in final)
  9.8 mL of 'buffer'

- 10 mL 'ATP' are prepared per assay :

  6.7 μl of 3 mM stock of ATP giving a final concentration of 1 μM per well 10 mL of 'buffer'

- 2.5 mL of each PI3K construct are prepared per assay in 'PI' with the following final concentration :

  10 nM PI3K alfa EMV B1075

  25 nM beta EMV BV949

  10 nM delta EMV BV1060

  150 nM gamma EMV BV950

- 5 μl of 'PI/PI3K' are added per well.
- 5 μl 'ATP' are added per well to start the reaction.
- The plates are then incubated at room temperature for 60 minutes (alfa, beta, delta) or 120 minutes (gamma).
- The reaction is terminated by the addition of 10 μl Kinase-Glo (Promega Cat. No. #6714).
- The assay plates are read after 10 minutes in Synergy 2 reader (BioTek, Vermont USA) with an integration time of 100 milliseconds and sensitivity set to 191.
- Output: The High control is around 60'000 counts and the Low control is 30'000 or lower
- This luminescence assay gives a useful Z' ratio between 0.4 and 0.7

The Z' value is a universal measurement of the robustness of an assay. A Z' between 0.5 and 1.0 is considered an excellent assay.

For this assay, the PI3K constructs mentioned are prepared as follows:

### 1.2 Generation of gene constructs

Two different constructs, BV 1052 and BV 1075, are used to generate the PI3 Kinase α proteins for compound screening.

<u>PI3Kα BV-1052 p85(iSH2)-Gly linker-p110a(D20aa)-C-term His tag</u>

PCR products for the inter SH2 domain (iSH2) of the p85 subunit and for the p110-a subunit (with a deletion of the first 20 amino acids) are generated and fused by overlapping PCR.

The iSH2 PCR product is generated from first strand cDNA using initially primers gwG130-p01 (5'-CGAGAATATGATAGATTATATGAAGAAT-3') (SEQ ID NO: 1) and gwG130-p02 (5'-TGGTTT-AATGCTGTTCATACGTTTGTCAAT-3') (SEQ ID NO: 2). Subsequently in a secondary PCR reaction, Gateway (Invitrogen AG, Basel, Switzerland) recombination AttB1 sites and linker sequences are added at the 5'end and 3'end of the p85 iSH2 fragment respectively, using primers gwG130-p03 (5'-GGGACAAGTTTGTACAAAAAAGCAGGCTACGAAGGAGATATACATAT-GCGAGAATATGATAGATTATATGAAGAAT -3') (SEQ ID NO: 3) and gwG152-p04 (5'- TACCATAATTCCACCACCACCACCGGAAATTCCCCCTGGTTT-AATGCTGTTCATACGTTTGTCAAT-3') (SEQ ID NO: 4).

The p110-a fragment is also generated from first strand cDNA, initially using primers gwG152-p01 (5'- CTAGTGGAATGTTTACTACCAAATGG-3') (SEQ ID NO: 5) and gwG152-p02 (5'- GTTCAATG-CATGCTGTTTAATTGTGT -3') (SEQ ID NO: 6).

In a subsequent PCR reaction, linker sequence and a Histidine tag are added at the 5'end and 3'end of the p110-a fragment respectively, using primers gw152-p03 (5'-GGGGGAATTTCCGGTGGTGGTGGTGGAATTATGGTAC-TAGTGGAATGTTTACTACC-AAATGGA-3') (SEQ ID NO: 7) and gwG152-p06 (5'-AGCTCCGTGATGGTGATGGTGATGTGCTCCGTTCAATG-CATGCTGTTTAATTGTGT-3') (SEQ ID NO: 8).

The p85-iSH2/p110-a fusion protein is assembled in a third PCR reaction by the overlapping linkers at the 3'end of the iSH2 fragment and the 5'end of the p110-a fragment, using the above mentioned gwG130-p03 primer and a primer containing an overlapping Histidine tag and the AttB2 recombination sequences (5'-GGGACCACTTTGTACAAGAAAGCTGGGTTTAAGCTCCGTGATGGTGATGGTGAT-GTGCTCC-3') (SEQ ID NO: 9).

This final product is recombined in a (Invitrogen) OR reaction into the donor vector pDONR201 to generate the ORF318 entry clone. This clone is verified by sequencing and used in a Gateway LR reaction to transfer the insert into the Gateway adapted pBlueBac4.5 (Invitrogen) vector for generation of the baculovirus expression vector LR410.

PI3Kα BV-1075 p85(iSH2)-12 XGly linker-p110a(D20aa)-C-term His tag

The construct for Baculovirus BV-1075 is generated by a three-part ligation comprised of a p85 fragment and a p110-a fragment cloned into vector pBlueBac4.5. The p85 fragment is derived from plasmid p1661-2 digested with Nhe/Spe. The p110-a fragment derived from LR410 (see above) as a SpeI/HindIII fragment. The cloning vector pBlueBac4.5 (Invitrogen) is digested with Nhe/HindIII. This results in the construct PED 153.8

The p85 component (iSH2) is generated by PCR using ORF 318 (described above) as a template and one forward primer KAC1028 (5'- GCTAGCATGCGAGAATATGATAGATTATATGAAGAATATACC) (SEQ ID NO: 10) and two reverse primers, KAC1029 (5'- GCCTCCACCACCTCCGCCTGGTTTAATGCTGTTCATACGTTTGTC) (SEQ ID NO: 11) and KAC1039 (5'-TACTAGTCCGCCTCCACCACCTCCGCCTCCACCACCTCCGCC) (SEQ ID NO: 12).

The two reverse primers overlap and incorporate the 12x Gly linker and the N-terminal sequence of the p110a gene to the SpeI site. The 12x Gly linker replaces the linker in the BV1052 construct. The PCR fragment is cloned into pCR2.1 TOPO (Invitrogen). Of the resulting clones, p1661-2 is determined to be correct. This plasmid is digested with Nhe and SpeI and the resulting fragment is gel-isolated and purified for sub-cloning.

The p110-a cloning fragment is generated by enzymatic digest of clone LR410 (see above) with Spe I and HindIII. The SpeI site is in the coding region of the p110a gene.

The resulting fragment is gel-isolated and purified for sub-cloning.

The cloning vector, pBlueBac4.5 (Invitrogen) is prepared by enzymatic digestion with Nhe and HindIII. The cut vector is purified with Qiagen (Quiagen N.V, Venlo, Netherlands) column and then dephosphorylated with Calf Intestine alkaline phosphatase (CIP) (New England BioLabs, Ipswich, MA). After completion of the CIP reaction the cut vector is again column purified to generate the final vector. A 3 part ligation is performed using Roche Rapid ligase and the vendor specifications.

PI3Kβ BV-949 p85(iSH2)-Gly linker-p110b(full-length)-C-term His tag

PCR products for the inter SH2 domain (iSH2) of the p85 subunit and for the full-length p110-b subunit are generated and fused by overlapping PCR.

The iSH2 PCR product is generated from first strand cDNA initially using primers gwG130-p01 (5'-CGAGAATATGATAGATTATATGAAGAAT-3') (SEQ ID NO: 1) and gwG130-p02 (5'-TGGTTT-AATGCTGTTCATACGTTTGTCAAT-3') (SEQ ID NO: 2). Subsequently, in a secondary PCR reaction Gateway (Invitrogen) recombination AttB1 sites and linker sequences are added at the 5'end and 3'end of the p85 iSH2 fragment respectively, using primers gwG130-p03 (5'- GGGACAAGTTTGTACAAAAAAGCAGGCTACGAAGGAGATA-TACATATGCGAGAATATGATAGATTATATGAAGAAT -3') (SEQ ID NO: 3) and gwG130-p05 (5'-ACTGAAGCATCCTCCTCCTCCTCCTCCTGGTTTAAT-GCTGTTCATACGTTTGTC-3') (SEQ ID NO: 13).

The p110-b fragment is also generated from first strand cDNA initially using primers gwG130-p04 (5'-ATTAAACCAGGAGGAGGAGGAGGAGGATGCTTCAGTTTCATAATGCC-TCCTGCT - 3') (SEQ ID NO: 4)

which contains linker sequences and the 5'end of p110-b and gwG130-p06 (5'-AGCTCCGTGATGGTGATGGTGATGTGCTCCAGATCTGTAGTCTTT-CCGAACTGTGTG -3') (SEQ ID NO: 14)
which contains sequences of the 3'end of p110-b fused to a Histidine tag.

The p85-iSH2/p110-b fusion protein is assembled by an overlapping PCR a reaction of the linkers at the 3'end of the iSH2 fragment and the 5'end of the p110-b fragment, using the above mentioned gwG130-p03 primer and a primer containing an overlapping Histidine tag and the AttB2 recombination sequences (5'-GGGACCACTTTGTACAAGAAAGCTGGGTTT-AAGCTCCGTGATGGTGATGGTGATGTGCTCC-3') (SEQ ID NO: 15).

This final product is recombined in a Gateway (Invitrogen) OR reaction into the donor vector pDONR201 to generate the ORF253 entry clone. This clone is verified by sequencing and used in a Gateway LR reaction to transfer the insert into the Gateway adapted pBlueBac4.5 (Invitrogen) vector for generation of the baculovirus expression vector LR280.

PI3Kδ BV-1060 p85(iSH2)-Gly linker-p110d(full-length)-C-term His tag

PCR products for the inter SH2 domain (iSH2) of the p85 subunit and for the full-length p110-d subunit are generated and fused by overlapping PCR.

The iSH2 PCR product is generated from first strand cDNA using initially primers gwG130-p01 (5'-CGAGAATATGATAGATTATATGAAGAAT-3') (SEQ ID NO: 1) and gwG130-p02 (5'-TGGTTT-AATGCTGTTCATACGTTTGTCAAT-3') (SEQ ID NO: 2). Subsequently, in a secondary PCR reaction Gateway (Invitrogen) recombination AttB1 sites and linker sequences are added at the 5'end and 3'end of the p85 iSH2 fragment respectively, using primers gwG130-p03 (5'- GGGACAAGTTTGTACAAAAAAGCAGGCTACGAAGGAGATATACAT-ATGCGAGAATATGATAGATTATATGAAGAAT -3') (SEQ ID NO: 3) and gwG154-p04 (5'- TCCTCCTCCTCCTCCTCCTGGTTTAATGCTGTTCATACGTTTGTC - 3') (SEQ ID NO: 16).

The p110-a fragment is also generated from first strand cDNA using initially primers gwG154-p01 (5'-ATGCCCCCTGGGGTGGACTGCCCCAT-3') (SEQ ID NO: 17) and gwG154-p02 (5'- CTACTG-CCTGTTGTCTTTGGACACGT -3') (SEQ ID NO: 18).

In a subsequent PCR reaction linker sequences and a Histidine tag is added at the 5'end and 3'end of the p110-d fragment respectively, using primers gw154-p03 (5'- ATTAAACCAGGAGGAGGAGGAGGAGGACCCCCTGGGGTGGAC-TGCCCCATGGA -3') (SEQ ID NO: 19) and gwG154-p06 (5'-AGCTCCGTGATGGTGAT-GGTGATGTGCT-CCCTGCCTGTTGTCTTTGGACACGTTGT -3') (SEQ ID NO: 20).

The p85-iSH2/p110-d fusion protein is assembled in a third PCR reaction by the overlapping linkers at the 3'end of the iSH2 fragment and the 5'end of the p110-d fragment, using the above mentioned gwG130-p03 primer and a primer containing an overlapping Histidine tag and the Gateway (Invitrogen) AttB2 recombination sequences (5'-GGGACCACTTTGTA-CAAGAAAGCTGGGTTT-AAGCTCCGTGATGGTGATGGTGATGTGCTCC-3') (SEQ ID NO: 21).

This final product is recombined in a Gateway (Invitrogen) OR reaction into the donor vector pDONR201 to generate the ORF319 entry clone. This clone is verified by sequencing and used in a Gateway LR reaction to transfer the insert into the Gateway adapted pBlueBac4.5 (Invitrogen) vector for generation of the baculovirus expression vector LR415.

PI3Kγ BV-950 p110g(D144aa)-C-term His tag

This construct is obtained from Roger Williams lab, MRC Laboratory of Molecular Biology, Cambridge, UK (November, 2003). Description of the construct in: Pacold M. E. et al. (2000) Cell 103, 931-943.

**1.3 Protein expression and purification**

Methods to generate recombinant baculovirus and protein for PI3K isoforms:

The pBlue-Bac4.5 (for a, b, and d isoforms) or pVL1393 (for g) plasmids containing the different PI3 kinase genes are co-transfected with BaculoGold WT genomic DNA (BD Biosciences, Franklin Lakes, NJ, USA) using methods recommended by the vendor.

Subsequently, the recombinant baculovirus obtained from the transfection is plaque-purified on Sf9 insect cells to yield several isolates expressing recombinant protein.

Positive clones are selected by anti-HIS or anti-isoform antibody western. For PI3K alpha and delta isoforms, a secondary plaque-purification is performed on the first clonal virus stocks of PI3K. Amplification of all baculovirus isolates is performed at low multiplicity of infection (moi) to generate high-titer, low passage stock for protein production. The baculoviruses are designated BV1052 (α) and BV1075 (α), BV949 (β), BV1060 (δ) and BV950 (γ).

Protein production involves infection (passage 3 or lower) of suspended Tn5 (Trichoplusia ni) or TiniPro (Expression Systems, LLC, Woodland, CA, USA) cells in protein-free media at moi of 2-10 for 39-48 hours in 2 l glass Erlenmyer flasks (110 rpm) or wave-bioreactors (22-25 rpm). Initially, 10 l working volume wave-bioreactors are seeded at a density of 3e5 cells/mL at half capacity (5L). The reactor is rocked at 15 rpm during the cell growth phase for 72 hours, supplemented with 5% oxygen mixed with air (0.2 l per minute). Immediately prior to infection, the wave-reactor cultures are analyzed for density, viability and diluted to approximately 1.5e6 cell/mL. 100-500 mL of high titer, low passage virus is added following 2-4 hours of additional culture. Oxygen is increased to 35% for the 39-48 hour infection period and rocking platform rpm increased to 25. During infection, cells are monitored by Vicell viability analyzer (Beckman Coulter, Inc, Fullerton, CA, USA) bioprocess for viability, diameter and density. Nova Bioanalyzer (NOVA Biomedical Corp., Waltham, MA, USA) readings of various parameters and metabolites (pH, $O_2$ saturation, glucose, etc.) are taken every 12-18 hours until harvest.

The wave-bioreactor cells are collected within 40 hours post infection. Cells are collected by centrifugation (4 degrees C at 1500 rpm), and subsequently maintained on ice during pooling of pellets for lysis and purification. Pellet pools are made with small amounts of cold, un-supplemented Grace's media (w/o protease inhibitors).

PI3K alpha Purification Protocol For HTS (BV1052)

PI3K alpha is purified in three chromatographic steps: immobilized metal affinity chromatography on a Ni Sepharose resin (GE Healthcare, belonging to General Electric Company, Fairfield, CT, USA), gel filtration utilizing a Superdex 200 26/60 column (GE Healthcare), and finally a cation exchange step on a SP-XL column (GE Healthcare). All buffers are chilled to 4°C and lysis is performed chilled on ice. Column fractionation is performed rapidly at room temperature.

Typically frozen insect cells are lysed in a hypertonic lysis buffer and applied to a prepared IMAC column. The resin is washed with 3-5 column volumes of lysis buffer, followed by 3-5 column volumes wash buffer containing 45 mM imidazole, and the target protein is then eluted with a buffer containing 250 mM imidazole. Fractions are analyzed by Coomassie stained SDS-PAGE gels, and fractions containing target protein are pooled and applied to a prepared GFC column. Fractions from the GFC column are analyzed by Coomassie stained SDS-PAGE gels, and fractions containing target protein are pooled. The pool from the GFC column is diluted into a low salt buffer and applied to a prepared SP-XL column. The column is washed with low salt buffer until a stable A280 baseline absorbance is achieved, and eluted using a 20 column volume gradient from 0 mM NaCl to 500 mM NaCl. Again, fractions from the SP-XL column are analyzed by Coomassie stained SDS-PAGE gels, and fractions containing the target protein are pooled. The final pool is dialyzed into a storage buffer containing 50% glycerol and stored at -20°C. The final pool is assayed for activity in a phosphoinosititol kinase assay.

PI3K beta Purification Protocol For HTS (BV949)

PI3K beta is purified in two chromatographic steps: immobilized metal affinity chromatography (IMAC) on a Ni Sepharose resin (GE Healthcare) and gel filtration (GFC) utilizing a Superdex 200 26/60 column (GE Healthcare). All buffers are chilled to 4°C and lysis is performed chilled on ice. Column fractionation is performed rapidly at room temperature.

Typically frozen insect cells are lysed in a hypertonic lysis buffer and applied to a prepared IMAC column. The resin is washed with 3-5 column volumes of lysis buffer, followed by 3-5 column volumes wash buffer containing 45 mM imidazole, and the target protein is then eluted with a buffer containing 250 mM imidazole. Fractions are analyzed by Coomassie stained SDS-PAGE gels, and fractions containing target protein are pooled and applied to a prepared GFC column. Fractions from the GFC column are analyzed by Coomassie stained SDS-PAGE gels, and fractions containing target protein are pooled. The final pool is dialyzed into a storage buffer containing 50% glycerol and stored at -20°C. The final pool is assayed for activity in the phosphoinostitol kinase assay.

PI3K gamma Purification Protocol For HTS (BV950)

PI3K gamma is purified in two chromatographic steps: immobilized metal affinity chromatography (IMAC) on a Ni Sepharose resin (GE Healthcare) and gel filtration (GFC) utilizing a Superdex 200 26/60 column (GE Healthcare). All buffers are chilled to 4°C and lysis is performed chilled on ice. Column fractionation is performed rapidly at room temperature. Typically frozen insect cells are lysed in a hypertonic lysis buffer and applied to a prepared IMAC column. The resin is washed with 3-5 column volumes of lysis buffer, followed by 3-5 column volumes wash buffer containing 45 mM imidazole, and the target protein is then eluted with a buffer containing 250 mM imidazole. Fractions are analyzed by Coomassie stained SDS-PAGE gels, and fractions containing target protein are pooled and applied to a prepared GFC column. Fractions from the GFC column are analyzed by Coomassie stained SDS-PAGE gels, and fractions containing target protein are pooled. The final pool is dialyzed into a storage buffer containing 50% glycerol and stored

at -20°C. The final pool is assayed for activity in the phosphoinostitol kinase assay.

PI3K delta Purification Protocol For HTS (BV1060)

PI3K delta is purified in three chromatographic steps: immobilized metal affinity chromatography on a Ni Sepharose resin (GE Healthcare), gel filtration utilizing a Superdex 200 26/60 column (GE Healthcare), and finally a anion exchange step on a Q-HP column (GE Healthcare). All buffers are chilled to 4°C and lysis is performed chilled on ice. Column fractionation is performed rapidly at room temperature. Typically frozen insect cells are lysed in a hypertonic lysis buffer and applied to a prepared IMAC column. The resin is washed with 3-5 column volumes of lysis buffer, followed by 3-5 column volumes wash buffer containing 45 mM imidazole, and the target protein is then eluted with a buffer containing 250 mM imidazole. Fractions are analyzed by Coomassie stained SDS-PAGE gels, and fractions containing the target protein are pooled and applied to a prepared GFC column. Fractions from the GFC column are analyzed by Coomassie stained SDS-PAGE gels, and fractions containing the target protein are pooled. The pool from the GFC column is diluted into a low salt buffer and applied to a prepared Q-HP column. The column is washed with low salt buffer until a stable A280 baseline absorbance is achieved, and eluted using a 20 column volume gradient from 0 mM NaCl to 500 mM NaCl. Again, fractions from the Q-HP column are analyzed by Coomassie stained SDS-PAGE gels, and fractions containing the target protein are pooled. The final pool is dialyzed into a storage buffer containing 50% glycerol and stored at -20°C. The final pool is assayed for activity in the phosphoinostitol kinase assay.
$IC_{50}$ is determined by a four parameter curve fitting routine that comes along with "excel fit". A four parameter logistic equation is used to calculate $IC_{50}$ values (IDBS XLfit) of the percentage inhibition of each compound at 8 concentrations (usually 10, 3.0, 1.0, 0.3, 0.1, 0.030, 0.010 and 0.003 μM). Alternatively, $IC_{50}$ values are calculated using idbsXLfit model 204, which is a 4 parameter logistic model.

Yet alternatively, for an ATP depletion assay, compounds to be tested are dissolved in DMSO and directly distributed into a white 384-well plate at 0.5 μl per well. To start the reaction, 10 μl of 10 nM PI3 kinase and 5 μg/mL 1-alpha-phosphatidylinositol (PI) are added into each well followed by 10 μl of 2 μM ATP. The reaction is performed until approx 50% of the ATP is depleted, and then stopped by the addition of 20 μl of Kinase-Glo solution (Promega Corp., Madison, WI, USA). The stopped reaction is incubated for 5 minutes and the remaining ATP is then detected via luminescence. $IC_{50}$ values are then determined.
In one embodiment of the present invention, the PI3K inhibitor, wherein said inhibitor has an inhibitory action on the PI3K isoform delta, wherein the range of activity, expressed as $IC_{50}$, in the enzymatic PI3K delta assay is from is between 1 nM and 500 nM.

In another embodiment of the present invention, the PI3K inhibitor, wherein said inhibitor has an inhibitory action on the PI3K isoform delta, wherein the range of activity, expressed as $IC_{50}$, in the enzymatic PI3K delta assay is from is between 1 nM and 100 nM.

In another embodiment of the present invention, the PI3K inhibitor, wherein said inhibitor has an inhibitory action on the PI3K isoform delta, wherein the range of activity, expressed as $IC_{50}$, in the enzymatic PI3K delta assay is from is between 0.5nM and 10 nM.

In one embodiment of the present invention, the PI3K inhibitor, wherein said inhibitor has an inhibitory action on the PI3K isoform delta, wherein the range of activity, expressed as $IC_{50}$, in the cellular PI3K delta assay is from is between 1 nM and 1000 nM.

In another embodiment of the present invention, the PI3K inhibitor, wherein said inhibitor has an inhibitory action on the PI3K isoform delta, wherein the range of activity, expressed as $IC_{50}$, in the cellular PI3K delta assay is from is between 1 nM and 500 nM.

In another embodiment of the present invention, the PI3K inhibitor, wherein said inhibitor has an inhibitory action on the PI3K isoform delta where the inhibitor shows a selectivity for the PI3K isoform delta over one or more of the other isoforms wherein this selectivity is at least 10 fold.

In another embodiment of the present invention, the PI3K inhibitor, wherein said inhibitor has an inhibitory action on the PI3K isoform delta where the inhibitor shows a selectivity for the PI3K isoform delta over one or more of the other isoforms wherein this selectivity is at least 20 fold.

In another embodiment of the present invention, the PI3K inhibitor, wherein said inhibitor has an inhibitory action on the PI3K isoform delta where the inhibitor shows a selectivity for the PI3K isoform delta over the different paralogs PI3K α and β, wherein this selectivity is at least 10 fold.

In another embodiment of the present invention, the PI3K inhibitor, wherein said inhibitor has an inhibitory action on the PI3K isoform delta where the inhibitor shows a selectivity for the PI3K isoform delta over the different paralogs PI3K α and β, wherein this selectivity is at least 20 fold.

In another embodiment of the present invention, the PI3K inhibitor, wherein said inhibitor has an inhibitory action on the PI3K isoform delta, wherein the range of activity, expressed as $IC_{50}$, in the cellular PI3K delta assay is from is between 1 nM and 500 nM and wherein said inhibitor has an inhibitory action on the PI3K isoform delta where

the inhibitor shows a selectivity for the PI3K isoform delta over the different paralogs PI3K α and β, wherein this selectivity is at least 10 fold.

In another embodiment of the present invention, the PI3K inhibitor, wherein said inhibitor has an inhibitory action on the PI3K isoform delta, wherein the range of activity, expressed as $IC_{50}$, in the cellular PI3K delta assay is from is between 1 nM and 500 nM and wherein said inhibitor has an inhibitory action on the PI3K isoform delta where the inhibitor shows a selectivity for the PI3K isoform delta over the different paralogs PI3K α and β, wherein this selectivity is at least 20 fold.

**2. Cellular assays**

**2.1 Phosphoinositide-3 kinase (PI3K)-mediated Akt 1/2 (S473) phosphorylation in Rat-1 cells**

Rat-1 cells stably overexpressing a myristoylated form of the catalytic subunit of human phosphoinositide-3 kinase (PI3K) alpha, beta or delta were plated in 384-well plates at a density of 7500 (PI3K alpha), 6200 (PI3K beta), or 4000 (PI3K delta) cells in 30ul complete growth medium (Dulbecco's modified Eagle's medium (DMEM high glucose) supplemented with 10% (v/v) fetal bovine serum, 1% (v/v) MEM non essential amino acids, 10mM HEPES, 2mM L-glutamine, 10 μg/mL puromycin and 1% (v/v) Penicillin/Streptomycin) and were incubated at 37%C / 5%$CO_2$ / 95% humidity for 24h. Compounds were diluted in 384-well compound plates to obtain 8-point serial dilutions for 40 test compounds in 90% DMSO, as well as 4 reference compounds plus 16 high controls and 16 low (inhibited) controls. Predilution plates were prepared by dispensing pipetting 250 nl of compound solutions into 384-well polypropylen plates using a Hummingwell nanoliter dispensor. Compounds were prediluted by the addition of 49.75 ul complete growth medium. 10ul of prediluted compound solution were transferred to the cell plate using a 384-well pipettor, resulting in a final DMSO concentration of 0.11%.

Cells were incubated for 1 h at 37%C / 5%$CO_2$ / 95% humidity. The supernatant was removed, the cells were lysed in 20ul of lysis buffer for AlphaScreen® *SureFire*® detection.

For detection of p-AKT(Ser473), the *SureFire*® p-Akt 1/2 (Ser473) Assay Kit (PerkinElmer, U.S.A) was used. 5ul of cell lysate was transferred to 384-well low volume Proxiplates for detection using a 384-well pipettor. Addition of AlphaScreen® *SureFire*® reagents was done according to the manufacturer's protocol. First, 5ul of reaction buffer plus activation buffer mix containing AlphaScreen® acceptor beads was added, the plate was sealed, and incubated on a plate shaker for 2 hours at room temperature. Second, 2ul of dilution buffer containing AlphaScreen® donor beads was added, and the plate was incubated on plate shaker as above for a further 2 hours. The plate was read on an AlphaScreen® compatible plate reader, using standard AlphaScreen® settings.

**2.2 Determination of murine B cell activation**

PI3Kδ has been recognized to modulate B cell function when cells are stimulated through the B cell receptor (BCR) (Okkenhaug et al. Science 297:1031 (2002). For assessing the inhibitory property of compounds on B cell activation, the upregulation of activation markers CD86 and CD69 on murine B cells derived from mouse spleen antibody is measured after stimulation with anti-IgM. CD69 is a well known activation marker for B and T cells (Sancho et al. Trends Immunol. 26:136 (2005). CD86 (also known as B7-2) is primarily expressed on antigen-presenting cells, including B cells. Resting B cells express CD86 at low levels, but upregulate it following stimulation of e.g. the BCR or IL-4 receptor. CD86 on a B cell interacts with CD28 on T cells. This interaction is required for optimal T cell activation and for the generation of an optimal IgG1 response (Carreno et al. Annu Rev Immunol. 20:29 (2002)).

Spleens from Balb/c mice are collected, splenocytes are isolated and washed twice with RPMI containing 10% foetal bovine serum (FBS), 10 mM HEPES, 100 Units/mL penicilline/streptomycine. RPMI supplemented in this way is subsequently referred to as medium. The cells are adjusted to 2.5 X $10^6$ cells/mL in medium and 200 μl cell suspension (5 x$10^6$cells) are added to the appropriate wells of 96 well plates.

Then the cells are stimulated by adding 50 μl anti-IgM mAb in medium (final concentration: 30 μg/mL). After incubation for 24 hours at 37°C, the cells are stained with the following antibody cocktails: anti-mouse CD86-FITC, anti-mouse CD69-PerCP-Cy5.5, anti-mouse CD19-PerCP for the assessment of B cells, and anti-mouse CD3-FITC, anti-mouse CD69-PE for the assessment of T cells (2 μl of each antibody/well). After one hour at room temperature (rt) in the dark the cells are transferred to 96

Deepwell plates. The cells are washed once with 1 mL PBS containing 2% FBS and after re-suspension in 200 μl the samples are analyzed on a FACS Calibur flow cytometer. Lymphocytes are gated in the FSC/SSC dot plot according to size and granularity and further analyzed for expression of CD19, CD3 and activation markers (CD86, CD69). Data are calculated from dot blots as percentage of cells positively stained for activation markers within the CD19+ or CD3+ population using BD CellQest Software.

For assessing the inhibitory property of compounds, compounds are first dissolved and diluted in DMSO followed

by a 1:50 dilution in medium. Splenocytes from Balb/c mice are isolated, re-suspended and transfered to 96 well plates as described above (200 μl/well). The diluted compounds or solvent are added to the plates (25 μl) and incubated at 37°C for 1 hour. Then the cultures are stimulated with 25 μl anti-IgM mAb/well (final concentration 30 μg/mL) for 24 hours at 37°C and stained with anti-mouse CD86-FITC and anti-mouse CD19-PerCP (2 μl of each antibody/well). CD86 expression on CD19 positive B cells is quantified by flow cytometry as described above.

**2.3 Determination of rat B cell activation**

PI3Kδ has been recognized to modulate B cell function when cells are stimulated through the B cell receptor (BCR) (Okkenhaug et al. Science 297:1031 (2002). For assessing the inhibitory property of compounds on B cell activation, the upregulation of activation markers CD86 on rat B cells derived from whole blood is measured after stimulation with anti-IgM and recombinant IL-4. The CD86 molecule (also known as B7-2) is primarily expressed on antigen-presenting cells, including B cells. Resting B cells express CD86 at low levels, but upregulate it following stimulation of e.g. the BCR or IL-4 receptor. CD86 on a B cell interacts with CD28 on T cells. This interaction is required for optimal T cell activation and for the generation of an optimal IgG1 response (Carreno et al. Annu Rev Immunol. 20:29 (2002)).

Collection of rat blood

Whole blood was collected from the abdominal aorta adult male Lewis rats (LEW/HanHsd) oby using a 10 ml syringe with hypodermic needle pre-coated with sodium heparin. Blood was transferred into 50 ml Falcon tubes and the anticoagulant concentration was adjusted to 100 U/ml.

Stimulation of rat B cells and treatment with specific inhibitor

For assessment of the *in vitro* effects of immunosuppressive drugs, heparinized blood was prediluted to 50% with medium. As medium served DMEM high glucose (Animed cat# 1-26F01-I) supplemented with 100 U/ml penicillin, 100 mg/ml streptomycin, 2 mM L-glutamin, 50 mg/ml dextran 40 and 5% fetal calf serum (FCS, Fetaclone I, Gibco #10270-106). Then, 190 μl prediluted blood was spiked with 10 μl of pre-diluted test compound in 96 well U-bottomed microtiter plates (Nunc) resulting in a 3-fold serial dilution with a concentration range from 20 to 0.0003 μM. Control wells were pretreated with DMSO to obtain a final concentration of 0.5% DMSO. Cultures were set up in duplicates, mixed well by agitation on a plate shaker (Heidolph Titramax 101; 30 sec, speed 900), pipetting up and down and agitated on the plate shaker again. Cultures were incubated at 37°C, 5% $CO_2$ for 1 hr. Then, 20 μl of polyclonal goat anti-rat IgM Ab (Serotec, cat# 302001) and 10 μl of diluted recombinant rIL-4 (Immunotools # 340085) were added to obtain final concentrations of 30 μg/ml and 5 ng/ml, respectively. Plates were mixed by agitation on a plate shaker as above and incubated for 24 hrs at 37°C, 5% $CO_2$.

Determination of B cell activation by flow cytometry

After incubation, 15 μl of a 25 mM EDTA solution was added per well and shaken for 15 min to detach adherent cells. For analysis of surface activation markers, samples were then stained with PE-Cy5-labeled anti-ratCD45RA (BD cat# 557015) to allow gating on B cells in FACS analysis. In addition, samples were stained with PE-labeled anti-rat CD86 (BD cat# 551396). All staining procedures were performed at rt for 30 min in the dark. After incubation, samples were transferred to 96-deep well V-bottomed microtiter plates (Corning # 396096) containing 2 ml/well of BD Lysing Solution (BD # 349202). After lysis of erythrocytes samples were washed with 2 ml of CellWASH (BD # 349524). Data was acquired on an LSRII or FACScalibur flow cytometer (BD Biosciences) using Cellquest Plus or DIVA (version 6.1.1) software, respectively. Lymphocytes were gated in the FSC/SSC dot blot according to size and granularity and further analyzed for expression of CD45RA and activation markers. Data were calculated from dot blots or histograms as percentage of cells positively stained for activation markers within the CD45RA+ population.

Statistical evaluation

The percentage inhibition of B cell activation after exposure to drug was calculated by the following formula:

$$\%\ \text{Inhibition} = 100 \times \frac{\text{stimulation without drug} - \text{stimulation with drug}}{\text{stimulation without drug} - \text{unstimulated}}$$

ORIGIN 7 software (OriginLab Corporation, Northampton, MA) was used for non-linear regression curve fitting.

The drug concentration resulting in 50% inhibition ($IC_{50}$) was obtained by fitting the Hill equation to inhibition data.

**2.4 Determination of TLR9-induced IL-6 in mouse splenocytes**

Preparation of single cell suspension from mouse spleen

Spleens were dissected from C57BL/6 mice immediately following euthanasia. Excess fat was trimmed from the spleens prior to mashing the spleen through a 0.4 μM cell strainer using a plunger from a 5 ml syringe. A single cell suspension was prepared and the volume was adjusted to 15 ml in a 50 ml Falcon tube using cold PBS. Cells were centrifuged at 1500 rpm for 5 minutes at 4°C degrees prior to removal of supernatant and re-suspension in 5 ml of red blood cell lysis buffer per spleen and incubation for 5 minutes at room temperature. Ice cold PBS (30 ml) was added to the cells prior to centrifugation at 1500 rpm for 5 minutes at 4°C. The supernatant was removed and the cells were washed twice with 40 ml of murine splenocyte culture media (MSCM). MSCM consisted of RPMI supplemented with 100 units/ml Penicillin and 100 μg/ml Streptomycin, 1 x nonessential amino acids, 1 mM Sodium Pyruvate, 0.05 mM β-mercaptoethanol, and 10% heatinactivated Fetal Bovine Serum (FBS). Cells were re-suspended in 10-20 ml of MSCM and counted using a Countess cell counter. Approximately $60x10^6$ splenocytes were obtained from a single C57BL/6 mouse spleen.

Stimulation of murine splenocytes and treatment with specific inhibitor

Splenocytes were plated at a final density of $2x10^5$ cells/well in a volume of 100 μl in 96 well flat bottomed plates and incubated in a humidified 37°C incubator for 2-4 hours. Afterwards, compounds to be tested were dispensed using an automated liquid handling machine using previously prepared compound stock plates. Stock plates consisted of compounds (in 90%/10% DMSO/$ddH_20$) arrayed in 8-10 point using 2- or 3-fold dilutions. The liquid handling machine dispensed 1 μl of each dilution from the previously prepared compound source plate into the appropriate destination well in the 96-well plate. The final starting concentration of the compounds in the cell culture was 10 μM. The final concentration of DMSO in the cell cultures was 0.5%. Cells were incubated with compounds for 1 hour prior to addition of TLR ligand. Then, a 10x $EC_{80}$ concentration of CpG1826 was added in a volume of 20 μl (for a final culture volume of 200 μl) whereupon cultures were incubated overnight in a humidified 37°C incubator.

Determination of Interleukin-6 by ELISA

After overnight culture, plates were centrifugated at 2000 rpm for 5 minutes at room temperature. Subsequently 150 μl of each culture was transferred to 96-well V-bottomed plates and IL-6 levels were measured using commercially available mouse IL-6 sandwich ELISA kit. Briefly, plates were coated overnight with the capture antibody prior to blocking for 1 hour with PBS/0.1% BSA. Samples and standards were added in a volume of 50 μl and the plate was incubated for 2 hours at room temperature. After removal of the standards/samples, the plate was washed using PBS/0.05% Tween prior to addition of 50 μl of the biotinylated detection antibody whereupon the plate was incubated for 2 hours at room temperature with agitation. Plates were washed again prior to addition of 50 μl streptavidin-horseradish peroxidase per well for 20 minutes. Following additional plate washes 50 μl TMB substrate was added to each well and plates were incubated for 20 minutes prior addition of 25 μl/well stop solution. IL-6 levels were measured using a SpectraMax 190 Plate Reader (450 nm) and analyzed using SoftMax Pro and GraphPad Prism software.

**2.5 Determination of TLR9-induced IFNalpha in human peripheral blood mononuclear cells (PBMC)**

Preparation of PBMC from fresh human blood

Human blood (ca. 75 ml) was collected in 10 S-Monovette tubes containing Heparin (S-Monovette 7.5 mL NH Heparin 16 IU/mL blood; Starstedt). Leucosep™ tubes (30 mL #227290; Greiner Bio-one) were prepared by addition of 15 ml lymphocyte separation medium LSM1077™ per tube (#J15-004; PAA Laboratories) and centrifugation for 30 sec at 1000g. Some 25 ml blood was transferred to Leucosep™ tubes following dilution with equal parts of PBS (without Ca2+/Mg2+; #14190-094). Samples were centrifuged at 800g for 20 min at 22 °C using an Eppendorf 5810R centrifuge without brake. The PBMC layer was carefully removed from plasma:separation medium interface and transferred into clean 50 ml tube. Cells were washed once by addition of PBS (up to 45 ml) and centrifuged (1400rpm, 10 min at 22 °C) with brake (set at speed 9) using an Eppendorf 5810R. Pelleted cells were carefully resuspended in Media (RPMI 1640+GlutaMAX-I, 0.05 mM 2-mercaptoethanol, 10 mM HEPES and 5% v/v FCS) and samples pooled. The medium components 2-mercaptoethanol (#31350-010; 50 mM), Hepes (#15630-056, 1M) and RPMI 1640 (1x) + GlutaMAX-I (#61870-010) were obtained from Gibco. FCS (#2-01F36-1) was obtained from Amimed. The PBMC were counted using

a Countess® Automated cell counter (sample was pre-diluted 1:10 in Media, prior to the addition of equal volume (10 μl) of Trypan Blue). Cells were diluted to 4 x $10^6$ cells/ml and seeded in 384-well plates (#353962; Becton Dickinson AG) to give a final volume of 25 μl (i.e. 1 x $10^5$ cells/well).

Stimulation of PBMC and treatment with specific inhibitor

Compounds were pre-diluted in 100% v/v DMSO (#41640-100mL; Sigma-Aldrich), followed by transfer in Media (to achieve a final DMSO concentration of 0.25%). Cells were treated with appropriate compound dilution (5 μl) or vehicle control (5 μl) and incubated for 30 min at 37 °C in a humidified incubator in air with 5% (v/v) $CO_2$. Cells were stimulated with CpG2216 (0.3 μM; #tlrl-hodna; Invivogen) or vehicle control (10 μl/well) and incubated for 20 h. Plates were briefly centrifuged (200 x g for 2 min at 22 °C) and supernatant samples (30 μl) removed for quantification of IFNα levels.

Quantification of IFNα using AlphaLisa technology

For quantification of IFNalpha the human interferon AlphaLISA Kit (#AL264F) from PerkinElmer was used. An antibody mix containing anti-IFNα acceptor beads (5 μg/ml final) and biotinylated antibody anti-IFNα (0.5 nM final) is prepared fresh and dispensed (5 μl) into 384-well Optiplates™ (#6007299; PerkinElmer). Dilution of known IFNα standards (human IFNα B (2b)) were prepared and together with cell supernatants (5 μl) were added to plates above. Plates were briefly centrifuged (pulse at 200g), covered with adhesive sealing film, vortexed and incubated 1 h at room temperature in the dark. Streptavidin-coated donor beads (20 μg/ml final) was prepared and added to each well (5 μl) in a dark lit area (light sensitive mix). Plates were incubated 30 min at room temperature (Pates must not be centrifuged or covered). After incubation, the plates were read with an EnVision™ multiplate reader equipped with the ALPHA option using the instrument's own "AlphaScreen standard settings" (e.g. total measurement time: 550 ms, Laser 680 nm excitation time: 180 ms, mirror: D640 as, emission filter: M570w, center wavelength 570 nm, bandwidth 100 nm, transmittance 75%). Data were collected for analysis and quantification of IFNα levels.

Data evaluation and analysis

Data were analysed using Excel XL fit 4.0 (Microsoft) with XLfit add-in (IDBS; version 4.3.2). Specific IFNα concentrations were determined following extrapolation to standard curves using human IFNα B (2b). Individual $IC_{50}$ values of compounds were determined by nonlinear regression after fitting of curves to the experimental data.

**3 Determination of antibody production to sheep red blood cells (SRBC).**

In brief, OFA rats were injected i.v. with sheep erythrocytes on d0 and treated orally on four consecutive days (d0 to d3) with the compounds under investigation. Spleen cell suspensions were prepared on d4 and lymphocytes were plated onto soft agar in presence of indicator cells (SRBC) and complement. Lysis of the indicator cells due to secretion of SRBC-specific antibody (predominantly of the IgM subclass) and presence of complement yielded plaques. The number of plaques per plate were counted and expressed as number of plaques per spleen.

Immunization: Groups of five female OFA rats were immunized on day 0 with $2x10^8$/ml SRBC (obtained from Laboratory Animal Services LAS, Novartis Pharma AG) in a volume of 0.5ml per rat by i.v. injection.
Compound treatment: Animals were treated with compound suspended in 0.5% CMC, 0.5%Tween80 in for 4 consecutive days (days 0, 1, 2 and 3) starting on the day of immunization. Compound was administered orally twice daily with 12 hours intervalls between doses in an application volume of 5 ml/kg body weight.

Preparation of spleen cell suspensions:

On day 4, animals were euthanized with $CO_2$. Spleens were removed, weighed, and deposited in plastic tubes containing 10 ml of cold (4 °C) Hank's balanced salt solution (HBSS; Gibco, pH 7.3, containing 1 mg Phenolred/100ml) for each rat spleen. Spleens were homogenized with a glass potter, left on ice for 5 minutes and 1 ml supernatant was transferred into a new tube. Cells were washed once in 4 ml HBSS then supernatants were discarded and pellets re-suspended in 1 ml of HBSS. Lymphocyte numbers per spleen were determined by automated cell counter and spleen cell suspensions were adjusted to a cell concentration of $30x10^6$/ml.

Plaque forming assay:

Soft agar petri dishes were prepared with 0.7% agarose (SERVA) in HBSS.

In addition, one ml of 0.7% agarose was prepared in plastic tubes and kept at 48°C in a water bath. Some 50 μl of a $30x10^6$/ml spleen cell suspension and 50 μl of SRBC at 40 x $10^8$/ml were added, mixed rapidly (Vortex) and poured onto the prepared agarose dishes. Petri dishes were slightly tilted to achieve even distribution of cell mixture on agarose layer. The dishes were left at room temperature for 15 minutes and were then incubated at 37°C for 60 minutes. Then, 1.4ml guinea pig complement (Harlan; 10%) was added and the incubation continued for another 60 minutes at 37 °C. SRBC-specific antibodies released by the plated-out B cells bound to the antigen (SRBC) in their vicinity. These antigen-antibody complexes activated complement and led to the lysis of the SRBC leaving a bright spot (plaque) within the red erythrocyte layer. Plaques were counted with a microscope.

The following formula for determination of inhibition of plaque formation was used:

$$\%Inhibition = C*100/V-100$$

with: V= mean number of plaques/spleen for vehicle group; C= mean number of plaques/spleen for compound treated group

References:

N.K. Jerne & A.A. Nordin (1963) Plaque formation in agar by single antibody-producing cells. Science 140:405.

N.K. Jerne, A.A. Nordin & C. Henry (1963) The agar plaque technique for recognizing antibody-producing cells. In: "Cell Bound Antibodies", B. Amos & H. Koprowski, Eds., Wistar Inst. Press, Philadelphia pp.109-125.


**Biological data**

**Enzymatic Assay**

| Example | PI3K alpha (uM) | PI3K delta (uM) |
|---|---|---|
| **F1** | 0.109 | 0.003 |

**Cellular Assays**

| Example | Cell PI3Kδ / IC50 [umol l-1] | RWB/IC50 CD86 [nmol l-1] |
|---|---|---|
| **F1** | 0.011 | 7 |

**Claims**

1. The anhydrous crystalline form of (1,1-dioxo-hexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]-pyrrolidin-1-yl}-methanone.

2. The anhydrous crystalline form according to claim 1, **characterized by** an X-Ray powder diffraction pattern comprising the following peaks given at degrees 2-Theta +/- 0.2 degrees: 9.1, 10.2, 11.9, 13.0, 17.1, 17.7, 18.7, 20.3, 20.8, 26.0, 26.7, 23.2, 24.1, 24.8, 29.3, 27.4, and 21.4.

3. The anhydrous crystalline form according to claim 1 having a X-ray diffraction spectrum as shown in Figure 1.

4. The anhydrous crystalline form according to claim 1 having a differential scanning calorimetry graph as shown in shown in Figure 2.

5. A form according to anyone of claims 1 to 4, for use as pharmaceutical.

6. A combination comprising a therapeutically effective amount of a form according to anyone of claims 1 to 4, and

one or more therapeutically active agents.

7. A form according to anyone of claims 1 to 4, for use in the treatment of a diseases or disorders which are mediated by the activity of the PI3K enzymes, preferably by the activity of the PI3Kδ isoform.

8. A pharmaceutical composition comprising a therapeutically effective amount of a form according to anyone of claims 1 to 4, and one or more pharmaceutically acceptable carriers.

## Patentansprüche

1. Wasserfreie kristalline Form von (1,1-Dioxohexahydro-1lambda*6*-thiopyran-4-yl)-{(S)-3-[1-(6-methoxy-5-methyl-pyridin-3-yl)-2,3-dihydro-1H-pyrido[3,4-b][1,4]oxazin-7-yloxy]pyrrolidin-1-yl}methanon.

2. Wasserfreie kristalline Form nach Anspruch 1, **gekennzeichnet durch** ein Röntgenpulverdiffraktogramm mit den folgenden, in Grad 2-Theta +/- 0,2 Grad angegebenen Peaks: 9,1, 10,2, 11,9, 13,0, 17,1, 17,7, 18,7, 20,3, 20,8, 26,0, 26,7, 23,2, 24, 1, 24,8, 29,3, 27,4 und 21,4.

3. Wasserfreie kristalline Form nach Anspruch 1 mit einem wie in Figur 1 gezeigten Röntgendiffraktionsspektrum.

4. Wasserfreie kristalline Form nach Anspruch 1 mit einem wie in Figur 2 gezeigten dynamischen Differenzkalorimetriediagramm.

5. Form nach einem der Ansprüche 1 bis 4 zur Verwendung als Pharmazeutikum.

6. Kombination, umfassend eine therapeutisch wirksame Menge einer Form nach einem der Ansprüche 1 bis 4 und einen oder mehrere therapeutische Wirkstoffe.

7. Form nach einem der Ansprüche 1 bis 4 zur Verwendung bei der Behandlung von Krankheiten bzw. Erkrankungen, die durch die Aktivität der PI3K-Enzyme, vorzugsweise durch die Aktivität der PI3Kδ-Isoform, vermittelt werden.

8. Pharmazeutische Zusammensetzung, umfassend eine therapeutisch wirksame Menge einer Form nach einem der Ansprüche 1 bis 4 und einen oder mehrere pharmazeutisch unbedenkliche Träger.

## Revendications

1. Forme cristalline anhydre de la (1,1-dioxo-hexahydro-1lambda*6*-thiopyran-4-yl)-{(S)-3-[1-(6-méthoxy-5-méthyl-pyridin-3-yl)-2,3-dihydro-1H-pyrido[3,4-b][1,4]oxazin-7-yloxy]-pyrrolidin-1-yl}-méthanone.

2. Forme cristalline anhydre selon la revendication 1, **caractérisée par** un diagramme de diffraction X de poudre comprenant les pics suivants, donnés en degrés 2-thêta +/- 0,2 degré : 9,1, 10,2, 11, 9, 13,0, 17,1, 17,7, 18,7, 20,3, 20,8, 26,0, 26,7, 23,2, 24,1, 24,8, 29,3, 27,4 et 21,4.

3. Forme cristalline anhydre selon la revendication 1, présentant un spectre de diffraction X illustré par la Figure 1.

4. Forme cristalline anhydre selon la revendication 1, présentant un graphique de calorimétrie différentielle à balayage comme illustré par la Figure 2.

5. Forme selon l'une quelconque des revendications 1 à 4 pour utilisation en tant que produit pharmaceutique.

6. Combinaison comprenant une quantité thérapeutiquement active d'une forme selon l'une quelconque des revendications 1 à 4, ainsi qu'un ou plusieurs agents thérapeutiquement actifs.

7. Forme selon l'une quelconque des revendications 1 à 4 pour utilisation dans le traitement de troubles ou de maladies faisant intervenir l'activité des enzymes PI3K, préférentiellement l'activité de l'isoforme PI3Kδ.

8. Composition pharmaceutique comprenant une quantité thérapeutiquement active d'une forme selon l'une quelconque des revendications 1 à 4 ainsi qu'un ou plusieurs vecteurs pharmaceutiquement acceptables.

**Figure 1**

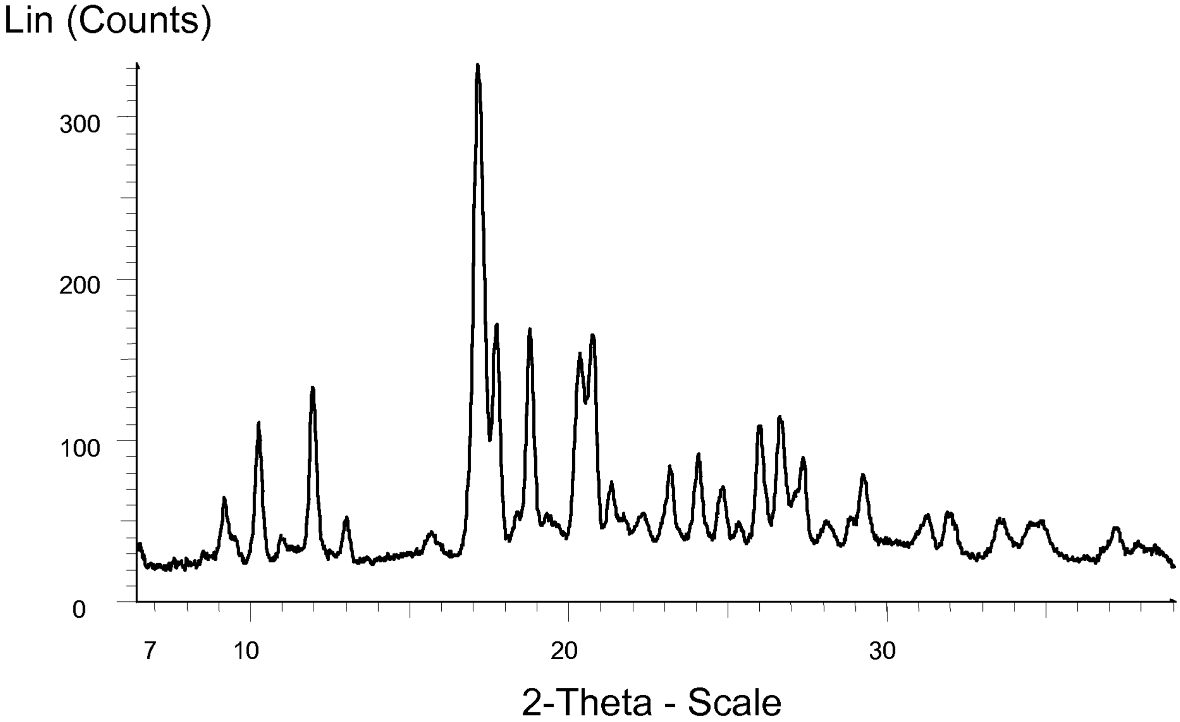


**Figure 2**

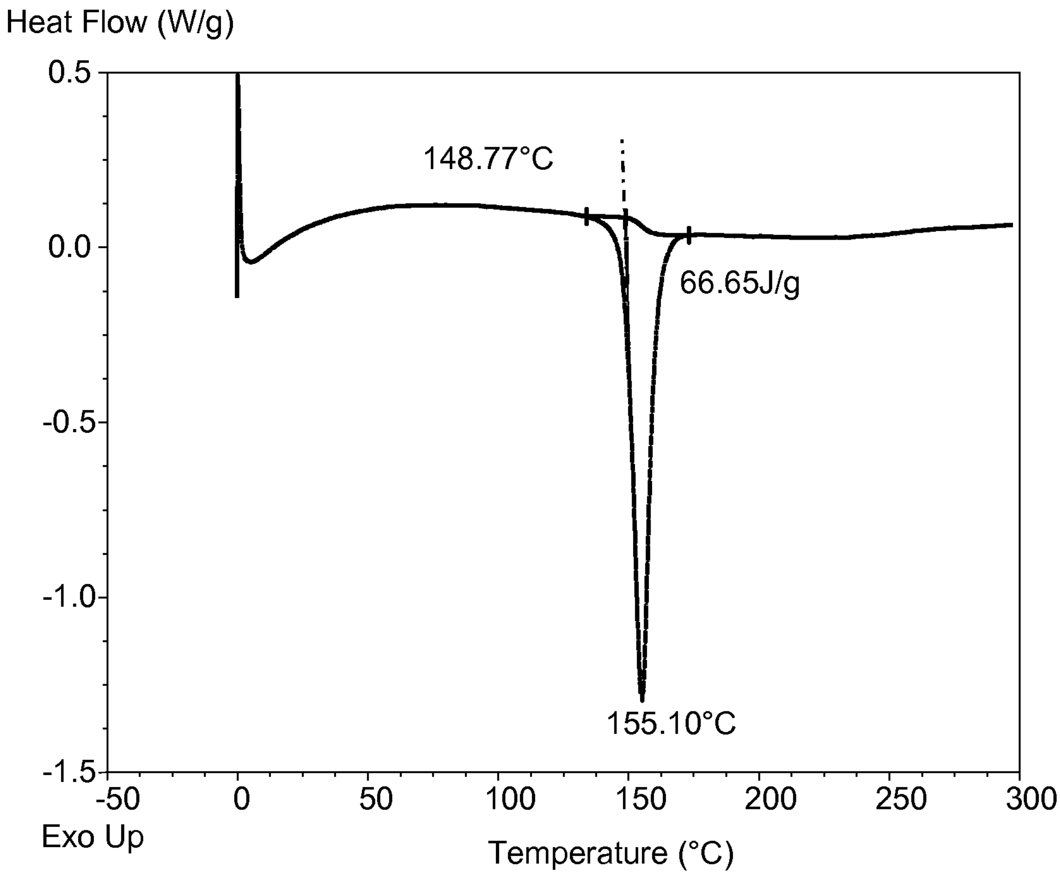

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO IB2012057554 W
- WO 2013093849 A
- WO 0238561 A
- WO 0382859 A
- WO 04052359 A
- WO 05066156 A
- US 4659516 A
- US 4636505 A
- US 4100274 A
- US 5843901 A
- WO 9917804 A
- US 5010099 A
- WO 9810121 A
- US 6194181 B
- WO 9825929 A
- WO 9808849 A
- WO 9943653 A
- WO 9822461 A
- WO 0031247 A
- WO 0222577 A
- WO 02092599 A
- US 5093330 A
- WO 0009495 A
- WO 9702266 A
- EP 0564409 A
- WO 9903854 A
- EP 0520722 A
- EP 0566226 A
- EP 0787722 A
- EP 0837063 A
- US 5747498 A
- WO 9810767 A
- WO 9730034 A
- WO 9749688 A
- WO 9738983 A
- WO 9630347 A
- WO 9633980 A
- WO 9503283 A
- WO 03013541 A
- WO 2006122806 A

### Non-patent literature cited in the description

- Remington's Pharmaceutical Sciences. Mack Printing Company, 1990, 1289-1329
- **HOUBEN-WEYL.** Methods of Organic Synthesis. Thieme, 1952, vol. 21
- *MRC Laboratory of Molecular Biology, Cambridge, UK,* November 2003
- **PACOLD M. E. et al.** *Cell,* 2000, vol. 103, 931-943
- **N.K. JERNE ; A.A. NORDIN.** Plaque formation in agar by single antibody-producing cells. *Science,* 1963, vol. 140, 405
- The agar plaque technique for recognizing antibody-producing cells. **N.K. JERNE ; A.A. NORDIN ; C. HENRY.** Cell Bound Antibodies. Wistar Inst. Press, 1963, 109-125